# EUROPÄISCHE PATENTANMELDUNG

(11) **EP 2 339 023 A2**
(43) Veröffentlichungstag der Anmeldung: **29.06.2011**
(21) Anmeldenummer: 10190882.0
(22) Anmeldetag: 11.11.2010
(51) Int. Cl.: C12Q 1/68

(54) **Nachweis von Mykobakterien und deren Resistenzgenen**

(30) Priorität: 13.11.2009 DE 102009054098
(71) Anmelder: Schöllhorn, Volkmar, Dr., 72479 Strassberg (DE)
(72) Erfinder: Schöllhorn, Volkmar, 72479 Strassberg (DE); Haid, Volker, 72336 Balingen (DE)
(74) Vertreter: Patentanwälte Ruff, Wilhelm, Beier, Dauster & Partner

(57) **Zusammenfassung**

Die Erfindung betrifft ein in vitro-Verfahren zum Nachweis von Tuberkulose-Erregern bzw. Mykobakterien bzw. deren DNA, umfassend die folgenden Schritte:
• Bereitstellen einer Probe, enthaltend Mykobakterien,
• Inkubieren der Probe mit einer Base,
• Isolieren von Mykobakterien-DNA aus den inkubierten Mykobakterien,
• Amplifizieren der isolierten Mykobakterien-DNA mittels Polymerase-Ketten-Reaktion (PCR), wobei mehr als 30 Amplifikationszyklen durchgeführt werden,
• Nachweisen der amplifizierten Mykobakterien-DNA.

Die Erfindung betrifft weiterhin Oligonukleotid-Primer, Oligonukleotid-Sonden, einen Träger mit Oligonukleotid-Sonden sowie einen Kit zum Nachweis von DNA von Tuberkulose-Erregern bzw. Mykobakterien bzw. deren DNA.

SEQUENCE LISTING

<110>  Volkmar Schöllhorn

<120>  Nachweis von Mykobakterien und deren Resistenzgenen

<130>  P49885DE

<160>  94

<170>  PatentIn version 3.3

<210>  1
<211>  20
<212>  DNA
<213>  künstliche Sequenz

<400>  1
caaatcgttt gtgcagaagg                                                    20


<210>  2
<211>  22
<212>  DNA
<213>  künstliche Sequenz

<400>  2
ctgaacttcg gtgttcttta gc                                                 22


<210>  3
<211>  20
<212>  DNA
<213>  künstliche Sequenz

<400>  3
atgaacgacg tcgaaacagc                                                    20


<210>  4
<211>  20
<212>  DNA
<213>  künstliche Sequenz
<400>  4
cgtagccgta caggatctcg                                                    20


<210>  5
<211>  19
<212>  DNA
<213>  künstliche Sequenz

<400>  5
agaaagggat ccgtcatgg                                                     19


<210>  6
<211>  20
<212>  DNA
<213>  künstliche Sequenz

<400>  6
actgaacggg atacgaatgg                                                    20

<210>  7
<211>  18
<212>  DNA
<213>  künstliche Sequenz

<400>  7
atgaccaccc aggacgtg                                                    18


<210>  8
<211>  19
<212>  DNA
<213>  künstliche Sequenz

<400>  8
gcacgctcac gtgacagac                                                   19


<210>  9
<211>  19
<212>  DNA
<213>  künstliche Sequenz

<400>  9
gaaggtgaag gtcggagtc                                                   19


<210>  10
<211>  20
<212>  DNA
<213>  künstliche Sequenz

<400>  10
gaagatggtg atgggatttc                                                  20


<210>  11
<211>  17
<212>  DNA
<213>  künstliche Sequenz

<400>  11
cgaactgtat gtcgtag                                                     17


<210>  12
<211>  17
<212>  DNA
<213>  künstliche Sequenz


<220>
<221>  modified_base
<222>  (7)..(7)
<223>  i

<400>  12
cgaactitat gtcgtag                                                     17


<210>  13

<211>  17
<212>  DNA
<213>  künstliche Sequenz

<220>
<221>  modified_base
<222>  (11)..(11)
<223>  i

<400>  13
cgaactgtat itcgtag                                                    17

<210>  14
<211>  15
<212>  DNA
<213>  künstliche Sequenz

<400>  14
gagacgatag gttgt                                                      15

<210>  15
<211>  15
<212>  DNA
<213>  künstliche Sequenz

<220>
<221>  modified_base
<222>  (3)..(3)
<223>  i

<400>  15
gaiacgatag gttgt                                                      15

<210>  16
<211>  15
<212>  DNA
<213>  künstliche Sequenz

<220>
<221>  modified_base
<222>  (6)..(6)
<223>  i

<400>  16
gagaciatag gttgt                                                      15

<210>  17
<211>  13
<212>  DNA
<213>  künstliche Sequenz

<400>  17
gatcaccagc ggc                                                        13

<210>  18

<211>  13
<212>  DNA
<213>  künstliche Sequenz

<220>
<221>  modified_base
<222>  (11)..(11)
<223>  i

<400>  18
gatcaccagc igc                                                          13

<210>  19
<211>  13
<212>  DNA
<213>  künstliche Sequenz

<220>
<221>  modified_base
<222>  (7)..(7)
<223>  i

<400>  19
gatcaciagc ggc                                                          13

<210>  20
<211>  14
<212>  DNA
<213>  künstliche Sequenz

<400>  20
ccaattcatg gacc                                                         14

<210>  21
<211>  14
<212>  DNA
<213>  künstliche Sequenz

<220>
<221>  modified_base
<222>  (14)..(14)
<223>  i

<400>  21
ccaattcatg gaci                                                         14

<210>  22
<211>  14
<212>  DNA
<213>  künstliche Sequenz

<220>
<221>  modified_base
<222>  (10)..(10)
<223>  i

<400>  22
ccaattcati gacc                                                          14

<210>  23
<211>  16
<212>  DNA
<213>  künstliche Sequenz

<400>  23
cggggttgac ccacaa                                                        16

<210>  24
<211>  16
<212>  DNA
<213>  künstliche Sequenz

<220>
<221>  modified_base
<222>  (10)..(10)
<223>  i

<400>  24
cggggttgai ccacaa                                                        16

<210>  25
<211>  16
<212>  DNA
<213>  künstliche Sequenz

<220>
<221>  modified_base
<222>  (14)..(14)
<223>  i

<400>  25
cggggttgac ccaiaa                                                        16

<210>  26
<211>  15
<212>  DNA
<213>  künstliche Sequenz

<400>  26
cgactgtcgg cgctg                                                         15

<210>  27
<211>  15
<212>  DNA
<213>  künstliche Sequenz

<220>
<221>  modified_base
<222>  (6)..(6)
<223>  i

<400>  27
cgactitcgg cgctg                                                        15

<210>  28
<211>  15
<212>  DNA
<213>  künstliche Sequenz

<220>
<221>  modified_base
<222>  (10)..(10)
<223>  i

<400>  28
cgactgtcgi cgctg                                                        15

<210>  29
<211>  15
<212>  DNA
<213>  künstliche Sequenz

<400>  29
gcagccgcgg taata                                                        15

<210>  30
<211>  15
<212>  DNA
<213>  künstliche Sequenz

<220>
<221>  modified_base
<222>  (6)..(6)
<223>  i

<400>  30
gcagcigcgg taata                                                        15

<210>  31
<211>  15
<212>  DNA
<213>  künstliche Sequenz

<220>
<221>  modified_base
<222>  (9)..(9)
<223>  i

<400>  31
gcagccgcig taata                                                        15

<210>  32
<211>  16
<212>  DNA
<213>  künstliche Sequenz

<400>  32
caccactccg aagaag                                                       16


<210>  33
<211>  16
<212>  DNA
<213>  künstliche Sequenz


<220>
<221>  modified_base
<222>  (13)..(13)
<223>  i

<400>  33
caccactccg aaiaag                                                       16


<210>  34
<211>  16
<212>  DNA
<213>  künstliche Sequenz


<220>
<221>  modified_base
<222>  (10)..(10)
<223>  i

<400>  34
caccactcci aagaag                                                       16


<210>  35
<211>  16
<212>  DNA
<213>  künstliche Sequenz

<400>  35
ccactccgaa gaagcc                                                       16


<210>  36
<211>  16
<212>  DNA
<213>  künstliche Sequenz


<220>
<221>  modified_base
<222>  (11)..(11)
<223>  i

<400>  36
ccactccgaa iaagcc                                                       16


<210>  37
<211>  16
<212>  DNA
<213>  künstliche Sequenz

<220>
<221>  modified_base
<222>  (8)..(8)
<223>  i

<400>  37
ccactcciaa gaagcc                                                       16


<210>  38
<211>  14
<212>  DNA
<213>  künstliche Sequenz

<400>  38
gggtgaagga cctg                                                         14


<210>  39
<211>  14
<212>  DNA
<213>  künstliche Sequenz


<220>
<221>  modified_base
<222>  (9)..(9)
<223>  i

<400>  39
gggtgaagia cctg                                                         14


<210>  40
<211>  14
<212>  DNA
<213>  künstliche Sequenz


<220>
<221>  modified_base
<222>  (5)..(5)
<223>  i

<400>  40
gggtiaagga cctg                                                         14


<210>  41
<211>  16
<212>  DNA
<213>  künstliche Sequenz

<400>  41
gtcacgtcat gaaagt                                                       16


<210>  42
<211>  16
<212>  DNA
<213>  künstliche Sequenz

<220>
<221>  modified_base
<222>  (6)..(6)
<223>  i

<400>  42
gtcacitcat gaaagt                                                        16


<210>  43
<211>  16
<212>  DNA
<213>  künstliche Sequenz


<220>
<221>  modified_base
<222>  (3)..(3)
<223>  i

<400>  43
gtiacgtcat gaaagt                                                        16


<210>  44
<211>  14
<212>  DNA
<213>  künstliche Sequenz

<400>  44
gtcacgtcat gaaa                                                          14


<210>  45
<211>  14
<212>  DNA
<213>  künstliche Sequenz


<220>
<221>  modified_base
<222>  (6)..(6)
<223>  i

<400>  45
gtcacitcat gaaa                                                          14


<210>  46
<211>  14
<212>  DNA
<213>  künstliche Sequenz


<220>
<221>  modified_base
<222>  (3)..(3)
<223>  i

<400>  46
gtiacgtcat gaaa                                                          14

<210>  47
<211>  16
<212>  DNA
<213>  künstliche Sequenz

<400>  47
gacgaagtcg taacaa                                                        16


<210>  48
<211>  16
<212>  DNA
<213>  künstliche Sequenz


<220>
<221>  modified_base
<222>  (7)..(7)
<223>  i

<400>  48
gacgaaitcg taacaa                                                        16


<210>  49
<211>  16
<212>  DNA
<213>  künstliche Sequenz


<220>
<221>  modified_base
<222>  (3)..(3)
<223>  i

<400>  49
gaigaagtcg taacaa                                                        16


<210>  50
<211>  14
<212>  DNA
<213>  künstliche Sequenz

<400>  50
gcgtcgatct acga                                                          14


<210>  51
<211>  14
<212>  DNA
<213>  künstliche Sequenz


<220>
<221>  modified_base
<222>  (5)..(5)
<223>  i

<400>  51
gcgtigatct acga                                                          14

<210>  52
<211>  14
<212>  DNA
<213>  künstliche Sequenz

<220>
<221>  modified_base
<222>  (3)..(3)
<223>  i

<400>  52
gcitcgatct acga                                                          14

<210>  53
<211>  15
<212>  DNA
<213>  künstliche Sequenz

<400>  53
cgtcgatcta cgaca                                                         15

<210>  54
<211>  15
<212>  DNA
<213>  künstliche Sequenz

<220>
<221>  modified_base
<222>  (4)..(4)
<223>  i

<400>  54
cgtigatcta cgaca                                                         15

<210>  55
<211>  15
<212>  DNA
<213>  künstliche Sequenz

<220>
<221>  modified_base
<222>  (8)..(8)
<223>  i

<400>  55
cgtcgatita cgaca                                                         15

<210>  56
<211>  15
<212>  DNA
<213>  künstliche Sequenz

<400>  56
ccaattcatg gtcca                                                         15

<210>  57
<211>  15
<212>  DNA
<213>  künstliche Sequenz

<220>
<221>  modified_base
<222>  (10)..(10)
<223>  i

<400>  57
ccaattcati gtcca                                                        15

<210>  58
<211>  15
<212>  DNA
<213>  künstliche Sequenz

<220>
<221>  modified_base
<222>  (14)..(14)
<223>  i

<400>  58
ccaattcatg gtcia                                                        15

<210>  59
<211>  14
<212>  DNA
<213>  künstliche Sequenz

<400>  59
ccaattcatg tacc                                                         14

<210>  60
<211>  14
<212>  DNA
<213>  künstliche Sequenz

<220>
<221>  modified_base
<222>  (13)..(13)
<223>  i

<400>  60
ccaattcatg taic                                                         14

<210>  61
<211>  14
<212>  DNA
<213>  künstliche Sequenz

<220>
<221>  modified_base

<222>  (10)..(10)
<223>  i

<400>  61
ccaattcati tacc                                                          14


<210>  62
<211>  17
<212>  DNA
<213>  künstliche Sequenz

<400>  62
ggggttgacc tacaagc                                                       17


<210>  63
<211>  17
<212>  DNA
<213>  künstliche Sequenz


<220>
<221>  modified_base
<222>  (13)..(13)
<223>  i

<400>  63
ggggttgacc taiaagc                                                       17


<210>  64
<211>  17
<212>  DNA
<213>  künstliche Sequenz


<220>
<221>  modified_base
<222>  (9)..(9)
<223>  i

<400>  64
ggggttgaic tacaagc                                                       17


<210>  65
<211>  16
<212>  DNA
<213>  künstliche Sequenz

<400>  65
cggggttgac cgacaa                                                        16


<210>  66
<211>  16
<212>  DNA
<213>  künstliche Sequenz


<220>
<221>  modified_base

<222>  (14)..(14)
<223>  i

<400>  66
cggggttgac cgaiaa                                                        16


<210>  67
<211>  16
<212>  DNA
<213>  künstliche Sequenz


<220>
<221>  modified_base
<222>  (10)..(10)
<223>  i

<400>  67
cggggttgai cgacaa                                                        16


<210>  68
<211>  16
<212>  DNA
<213>  künstliche Sequenz

<400>  68
cggggttgac ccgcaa                                                        16


<210>  69
<211>  16
<212>  DNA
<213>  künstliche Sequenz


<220>
<221>  modified_base
<222>  (14)..(14)
<223>  i

<400>  69
cggggttgac ccgiaa                                                        16


<210>  70
<211>  16
<212>  DNA
<213>  künstliche Sequenz


<220>
<221>  modified_base
<222>  (11)..(11)
<223>  i

<400>  70
cggggttgac icgcaa                                                        16


<210>  71
<211>  15

<212>  DNA
<213>  künstliche Sequenz

<400>  71
cgactgttgg cgctg                                                        15


<210>  72
<211>  15
<212>  DNA
<213>  künstliche Sequenz


<220>
<221>  modified_base
<222>  (10)..(10)
<223>  i

<400>  72
cgactgttgi cgctg                                                        15


<210>  73
<211>  15
<212>  DNA
<213>  künstliche Sequenz


<220>
<221>  modified_base
<222>  (6)..(6)
<223>  i

<400>  73
cgactittgg cgctg                                                        15


<210>  74
<211>  15
<212>  DNA
<213>  künstliche Sequenz

<400>  74
cgactgtggg cgctg                                                        15


<210>  75
<211>  15
<212>  DNA
<213>  künstliche Sequenz


<220>
<221>  modified_base
<222>  (10)..(10)
<223>  i

<400>  75
cgactgtggi cgctg                                                        15


<210>  76
<211>  15

<212>  DNA
<213>  künstliche Sequenz

<220>
<221>  modified_base
<222>  (6)..(6)
<223>  i

<400>  76
cgactitggg cgctg                                                        15

<210>  77
<211>  13
<212>  DNA
<213>  künstliche Sequenz

<400>  77
gatcaccacc ggc                                                          13

<210>  78
<211>  13
<212>  DNA
<213>  künstliche Sequenz

<220>
<221>  modified_base
<222>  (11)..(11)
<223>  i

<400>  78
gatcaccacc igc                                                          13

<210>  79
<211>  13
<212>  DNA
<213>  künstliche Sequenz

<220>
<221>  modified_base
<222>  (7)..(7)
<223>  i

<400>  79
gatcaciacc ggc                                                          13

<210>  80
<211>  14
<212>  DNA
<213>  künstliche Sequenz

<400>  80
gatcaccaca ggca                                                         14

<210>  81
<211>  14

<212>  DNA
<213>  künstliche Sequenz

<220>
<221>  modified_base
<222>  (12)..(12)
<223>  i

<400>  81
gatcaccaca gica                                                          14

<210>  82
<211>  14
<212>  DNA
<213>  künstliche Sequenz

<220>
<221>  modified_base
<222>  (7)..(7)
<223>  i

<400>  82
gatcaciaca ggca                                                          14

<210>  83
<211>  15
<212>  DNA
<213>  künstliche Sequenz

<400>  83
gaggcgatag gttgt                                                         15

<210>  84
<211>  15
<212>  DNA
<213>  künstliche Sequenz

<220>
<221>  modified_base
<222>  (6)..(6)
<223>  i

<400>  84
gaggciatag gttgt                                                         15

<210>  85
<211>  15
<212>  DNA
<213>  künstliche Sequenz

<220>
<221>  modified_base
<222>  (3)..(3)
<223>  i

<400>  85
gaigcgatag gttgt                                                        15


<210>  86
<211>  15
<212>  DNA
<213>  künstliche Sequenz

<400>  86
gagatgatag gttgt                                                        15


<210>  87
<211>  15
<212>  DNA
<213>  künstliche Sequenz


<220>
<221>  modified_base
<222>  (6)..(6)
<223>  i

<400>  87
gagatiatag gttgt                                                        15


<210>  88
<211>  15
<212>  DNA
<213>  künstliche Sequenz


<220>
<221>  modified_base
<222>  (3)..(3)
<223>  i

<400>  88
gaiatgatag gttgt                                                        15


<210>  89
<211>  15
<212>  DNA
<213>  künstliche Sequenz

<400>  89
agacgatagg ctgtc                                                        15


<210>  90
<211>  15
<212>  DNA
<213>  künstliche Sequenz


<220>
<221>  modified_base
<222>  (13)..(13)
<223>  i

<400>  90
agacgatagg ctitc                                                        15

<210>  91
<211>  15
<212>  DNA
<213>  künstliche Sequenz

<220>
<221>  modified_base
<222>  (9)..(9)
<223>  i

<400>  91
agacgataig ctgtc                                                        15

<210>  92
<211>  15
<212>  DNA
<213>  künstliche Sequenz

<400>  92
gagacgatag gatgt                                                        15

<210>  93
<211>  15
<212>  DNA
<213>  künstliche Sequenz

<220>
<221>  modified_base
<222>  (14)..(14)
<223>  i

<400>  93
gagacgatag gatit                                                        15

<210>  94
<211>  15
<212>  DNA
<213>  künstliche Sequenz

<220>
<221>  modified_base
<222>  (10)..(10)
<223>  i

<400>  94
gagacgatai gatgt                                                        15

SEQUENCE LISTING

<110> Volkmar Schöllhorn

<120> Nachweis von Mykobakterien und deren Resistenzgenen

<130> P49885DE

<140> EP10190882.0-1222

<141> 2010-11-11

<160> 94

<170> PatentIn version 3.3

<210> 1
<211> 20
<212> DNA
<213> künstliche Sequenz

<220>
<221> misc_feature
<222> (1)..(20)
<223> Oligonukleotid-Primer

<400> 1
caaatcgttt gtgcagaagg 20

<210> 2
<211> 22
<212> DNA
<213> künstliche Sequenz

<220>
<221> misc_feature
<222> (1)..(22)
<223> Oligonukleotid-Primer

<400> 2
ctgaacttcg gtgttcttta gc 22

<210> 3
<211> 20
<212> DNA
<213> künstliche Sequenz

<220>
<221> misc_feature
<222> (1)..(20)
<223> Oligonukleotid-Primer

<400> 3
atgaacgacg tcgaaacagc 20

<210> 4
<211> 20
<212> DNA
<213> künstliche Sequenz

<220>

<221>  misc_feature
<222>  (1)..(20)
<223>  Oligonukleotid-Primer

<400>  4
cgtagccgta caggatctcg                                                    20


<210>  5
<211>  19
<212>  DNA
<213>  künstliche Sequenz


<220>
<221>  misc_feature
<222>  (1)..(19)
<223>  Oligonukleotid-Primer

<400>  5
agaaagggat ccgtcatgg                                                     19


<210>  6
<211>  20
<212>  DNA
<213>  künstliche Sequenz


<220>
<221>  misc_feature
<222>  (1)..(20)
<223>  Oligonukleotid-Primer

<400>  6
actgaacggg atacgaatgg                                                    20


<210>  7
<211>  18
<212>  DNA
<213>  künstliche Sequenz


<220>
<221>  misc_feature
<222>  (1)..(18)
<223>  Oligonukleotid-Primer

<400>  7
atgaccaccc aggacgtg                                                      18


<210>  8
<211>  19
<212>  DNA
<213>  künstliche Sequenz


<220>
<221>  misc_feature
<222>  (1)..(19)
<223>  Oligonukleotid-Primer

<400>  8
gcacgctcac gtgacagac                                                     19

<210>  9
<211>  19
<212>  DNA
<213>  künstliche Sequenz


<220>
<221>  misc_feature
<222>  (1)..(19)
<223>  Oligonukleotid-Primer

<400>  9
gaaggtgaag gtcggagtc                                                    19


<210>  10
<211>  20
<212>  DNA
<213>  künstliche Sequenz


<220>
<221>  misc_feature
<222>  (1)..(20)
<223>  Oligonukleotid-Primer

<400>  10
gaagatggtg atgggatttc                                                   20


<210>  11
<211>  17
<212>  DNA
<213>  künstliche Sequenz


<220>
<221>  misc_feature
<222>  (1)..(17)
<223>  Oligonukleotid-Sonde

<400>  11
cgaactgtat gtcgtag                                                      17


<210>  12
<211>  17
<212>  DNA
<213>  künstliche Sequenz


<220>
<221>  misc_feature
<222>  (1)..(17)
<223>  Oligonukleotid-Sonde

<220>
<221>  modified_base
<222>  (7)..(7)
<223>  i

<400>  12
cgaactitat gtcgtag                                                      17


<210>  13
<211>  17
<212>  DNA

<213>  künstliche Sequenz

<220>
<221>  misc_feature
<222>  (1)..(17)
<223>  Oligonukleotid-Sonde

<220>
<221>  modified_base
<222>  (11)..(11)
<223>  i

<400>  13
cgaactgtat itcgtag                                                    17

<210>  14
<211>  15
<212>  DNA
<213>  künstliche Sequenz

<220>
<221>  misc_feature
<222>  (1)..(15)
<223>  Oligonukleotid-Sonde

<400>  14
gagacgatag gttgt                                                      15

<210>  15
<211>  15
<212>  DNA
<213>  künstliche Sequenz

<220>
<221>  misc_feature
<222>  (1)..(15)
<223>  Oligonukleotid-Sonde

<220>
<221>  modified_base
<222>  (3)..(3)
<223>  i

<400>  15
gaiacgatag gttgt                                                      15

<210>  16
<211>  15
<212>  DNA
<213>  künstliche Sequenz

<220>
<221>  misc_feature
<222>  (1)..(15)
<223>  Oligonukleotid-Sonde

<220>
<221>  modified_base
<222>  (6)..(6)
<223>  i

<400>  16
gagaciatag gttgt                                                        15


<210>  17
<211>  13
<212>  DNA
<213>  künstliche Sequenz


<220>
<221>  misc_feature
<222>  (1)..(13)
<223>  Oligonukleotid-Sonde

<400>  17
gatcaccagc ggc                                                          13


<210>  18
<211>  13
<212>  DNA
<213>  künstliche Sequenz


<220>
<221>  misc_feature
<222>  (1)..(13)
<223>  Oligonukleotid-Sonde

<220>
<221>  modified_base
<222>  (11)..(11)
<223>  i

<400>  18
gatcaccagc igc                                                          13


<210>  19
<211>  13
<212>  DNA
<213>  künstliche Sequenz


<220>
<221>  misc_feature
<222>  (1)..(13)
<223>  Oligonukleotid-Sonde

<220>
<221>  modified_base
<222>  (7)..(7)
<223>  i

<400>  19
gatcaciagc ggc                                                          13


<210>  20
<211>  14
<212>  DNA
<213>  künstliche Sequenz


<220>
<221>  misc_feature
<222>  (1)..(14)

<223>  Oligonukleotid-Sonde

<400>  20
ccaattcatg gacc                                                          14


<210>  21
<211>  14
<212>  DNA
<213>  künstliche Sequenz


<220>
<221>  misc_feature
<222>  (1)..(14)
<223>  Oligonukleotid-Sonde

<220>
<221>  modified_base
<222>  (14)..(14)
<223>  i

<400>  21
ccaattcatg gaci                                                          14


<210>  22
<211>  14
<212>  DNA
<213>  künstliche Sequenz


<220>
<221>  misc_feature
<222>  (1)..(14)
<223>  Oligonukleotid-Sonde

<220>
<221>  modified_base
<222>  (10)..(10)
<223>  i

<400>  22
ccaattcati gacc                                                          14


<210>  23
<211>  16
<212>  DNA
<213>  künstliche Sequenz


<220>
<221>  misc_feature
<222>  (1)..(16)
<223>  Oligonukleotid-Sonde

<400>  23
cggggttgac ccacaa                                                        16


<210>  24
<211>  16
<212>  DNA
<213>  künstliche Sequenz


<220>

<221>  misc_feature
<222>  (1)..(16)
<223>  Oligonukleotid-Sonde

<220>
<221>  modified_base
<222>  (10)..(10)
<223>  i

<400>  24
cggggttgai ccacaa                                                    16


<210>  25
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (14)..(14)
<223>  i


<400>  25
cggggttgac ccaiaa                                                    16


<210>  26
<211>  15
<212>  DNA
<213>  künstliche Sequenz


<220>
<221>  misc_feature
<222>  (1)..(15)
<223>  Oligonukleotid-Sonde

<400>  26
cgactgtcgg cgctg                                                     15


<210>  27
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (6)..(6)
<223>  i

<400>  27
cgactitcgg cgctg                                                     15

<210>  28
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (10)..(10)
<223>  i

<400>  28
cgactgtcgi cgctg                                                    15


<210>  29
<211>  15
<212>  DNA
<213>  künstliche Sequenz


<220>
<221>  misc_feature
<222>  (1)..(15)
<223>  Oligonukleotid-Sonde

<400>  29
gcagccgcgg taata                                                    15


<210>  30
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (6)..(6)
<223>  i

<400>  30
gcagcigcgg taata                                                    15


<210>  31
<211>  15
<212>  DNA
<213>  künstliche Sequenz


<220>
<221>  misc_feature
<222>  (1)..(15)
<223>  Oligonukleotid-Sonde

<220>

<221>  modified_base
<222>  (9)..(9)
<223>  i

<400>  31
gcagccgcig taata                                                    15


<210>  32
<211>  16
<212>  DNA
<213>  künstliche Sequenz


<220>
<221>  misc_feature
<222>  (1)..(16)
<223>  Oligonukleotid-Sonde

<400>  32
caccactccg aagaag                                                   16


<210>  33
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (13)..(13)
<223>  i

<400>  33
caccactccg aaiaag                                                   16


<210>  34
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (10)..(10)
<223>  i

<400>  34
caccactcci aagaag                                                   16


<210>  35
<211>  16
<212>  DNA
<213>  künstliche Sequenz

<220>
<221>  misc_feature
<222>  (1)..(16)
<223>  Oligonukleotid-Sonde

<400>  35
ccactccgaa gaagcc                                                    16


<210>  36
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (11)..(11)
<223>  i

<400>  36
ccactccgaa iaagcc                                                    16


<210>  37
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (8)..(8)
<223>  i

<400>  37
ccactcciaa gaagcc                                                    16


<210>  38
<211>  14
<212>  DNA
<213>  künstliche Sequenz


<220>
<221>  misc_feature
<222>  (1)..(14)
<223>  Oligonukleotid-Sonde

<400>  38
gggtgaagga cctg                                                      14


<210>  39
<211>  14
<212>  DNA

<213>  künstliche Sequenz

<220>
<221>  misc_feature
<222>  (1)..(14)
<223>  Oligonukleotid-Sonde

<220>
<221>  modified_base
<222>  (9)..(9)
<223>  i

<400>  39
gggtgaagia cctg                                                          14

<210>  40
<211>  14
<212>  DNA
<213>  künstliche Sequenz

<220>
<221>  misc_feature
<222>  (1)..(14)
<223>  Oligonukleotid-Sonde

<220>
<221>  modified_base
<222>  (5)..(5)
<223>  i

<400>  40
gggtiaagga cctg                                                          14

<210>  41
<211>  16
<212>  DNA
<213>  künstliche Sequenz

<220>
<221>  misc_feature
<222>  (1)..(16)
<223>  Oligonukleotid-Sonde

<400>  41
gtcacgtcat gaaagt                                                        16

<210>  42
<211>  16
<212>  DNA
<213>  künstliche Sequenz

<220>
<221>  misc_feature
<222>  (1)..(16)
<223>  Oligonukleotid-Sonde

<220>
<221>  modified_base
<222>  (6)..(6)
<223>  i

<400>  42
gtcacitcat gaaagt                                                    16


<210>  43
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (3)..(3)
<223>  i

<400>  43
gtiacgtcat gaaagt                                                    16


<210>  44
<211>  14
<212>  DNA
<213>  künstliche Sequenz


<220>
<221>  misc_feature
<222>  (1)..(14)
<223>  Oligonukleotid-Sonde

<400>  44
gtcacgtcat gaaa                                                      14


<210>  45
<211>  14
<212>  DNA
<213>  künstliche Sequenz


<220>
<221>  misc_feature
<222>  (1)..(14)
<223>  Oligonukleotid-Sonde

<220>
<221>  modified_base
<222>  (6)..(6)
<223>  i

<400>  45
gtcacitcat gaaa                                                      14


<210>  46
<211>  14
<212>  DNA
<213>  künstliche Sequenz


<220>
<221>  misc_feature
<222>  (1)..(14)

<223>  Oligonukleotid-Sonde

<220>
<221>  modified_base
<222>  (3)..(3)
<223>  i

<400>  46
gtiacgtcat gaaa                                                          14


<210>  47
<211>  16
<212>  DNA
<213>  künstliche Sequenz


<220>
<221>  misc_feature
<222>  (1)..(16)
<223>  Oligonukleotid-Sonde

<400>  47
gacgaagtcg taacaa                                                        16


<210>  48
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (7)..(7)
<223>  i

<400>  48
gacgaaitcg taacaa                                                        16


<210>  49
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (3)..(3)
<223>  i

<400>  49
gaigaagtcg taacaa                                                        16


<210>  50
<211>  14

<212>  DNA
<213>  künstliche Sequenz


<220>
<221>  misc_feature
<222>  (1)..(14)
<223>  Oligonukleotid-Sonde

<400>  50
gcgtcgatct acga                                                          14


<210>  51
<211>  14
<212>  DNA
<213>  künstliche Sequenz


<220>
<221>  misc_feature
<222>  (1)..(14)
<223>  Oligonukleotid-Sonde

<220>
<221>  modified_base
<222>  (5)..(5)
<223>  i

<400>  51
gcgtigatct acga                                                          14


<210>  52
<211>  14
<212>  DNA
<213>  künstliche Sequenz


<220>
<221>  misc_feature
<222>  (1)..(14)
<223>  Oligonukleotid-Sonde

<220>
<221>  modified_base
<222>  (3)..(3)
<223>  i

<400>  52
gcitcgatct acga                                                          14


<210>  53
<211>  15
<212>  DNA
<213>  künstliche Sequenz


<220>
<221>  misc_feature
<222>  (1)..(15)
<223>  Oligonukleotid-Sonde

<400>  53
cgtcgatcta cgaca                                                         15

<210>  54
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (4)..(4)
<223>  i

<400>  54
cgtigatcta cgaca                                                        15


<210>  55
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (8)..(8)
<223>  i

<400>  55
cgtcgatita cgaca                                                        15


<210>  56
<211>  15
<212>  DNA
<213>  künstliche Sequenz


<220>
<221>  misc_feature
<222>  (1)..(15)
<223>  Oligonukleotid-Sonde

<400>  56
ccaattcatg gtcca                                                        15


<210>  57
<211>  15
<212>  DNA
<213>  künstliche Sequenz


<220>
<221>  misc_feature
<222>  (1)..(15)
<223>  Oligonukleotid-Sonde

<220>
<221>  modified_base

<222>  (10)..(10)
<223>  i

<400>  57
ccaattcati gtcca                                                        15


<210>  58
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (14)..(14)
<223>  i

<400>  58
ccaattcatg gtcia                                                        15


<210>  59
<211>  14
<212>  DNA
<213>  künstliche Sequenz


<220>
<221>  misc_feature
<222>  (1)..(14)
<223>  Oligonukleotid-Sonde

<400>  59
ccaattcatg tacc                                                         14


<210>  60
<211>  14
<212>  DNA
<213>  künstliche Sequenz


<220>
<221>  misc_feature
<222>  (1)..(14)
<223>  Oligonukleotid-Sonde

<220>
<221>  modified_base
<222>  (13)..(13)
<223>  i

<400>  60
ccaattcatg taic                                                         14


<210>  61
<211>  14
<212>  DNA
<213>  künstliche Sequenz

<220>
<221>  misc_feature
<222>  (1)..(14)
<223>  Oligonukleotid-Sonde

<220>
<221>  modified_base
<222>  (10)..(10)
<223>  i

<400>  61
ccaattcati tacc                                                          14


<210>  62
<211>  17
<212>  DNA
<213>  künstliche Sequenz


<220>
<221>  misc_feature
<222>  (1)..(17)
<223>  Oligonukleotid-Sonde

<400>  62
ggggttgacc tacaagc                                                       17


<210>  63
<211>  17
<212>  DNA
<213>  künstliche Sequenz


<220>
<221>  misc_feature
<222>  (1)..(17)
<223>  Oligonukleotid-Sonde

<220>
<221>  modified_base
<222>  (13)..(13)
<223>  i

<400>  63
ggggttgacc taiaagc                                                       17


<210>  64
<211>  17
<212>  DNA
<213>  künstliche Sequenz


<220>
<221>  misc_feature
<222>  (1)..(17)
<223>  Oligonukleotid-Sonde

<220>
<221>  modified_base
<222>  (9)..(9)
<223>  i

<400>  64
ggggttgaic tacaagc                                                       17

<210>  65
<211>  16
<212>  DNA
<213>  künstliche Sequenz


<220>
<221>  misc_feature
<222>  (1)..(16)
<223>  Oligonukleotid-Sonde

<400>  65
cggggttgac cgacaa                                                    16


<210>  66
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (14)..(14)
<223>  i

<400>  66
cggggttgac cgaiaa                                                    16


<210>  67
<211>  16
<212>  DNA
<213>  künstliche Sequenz


<220>
<221>  misc_feature
<222>  (1)..(16)
<223>  Oligonukleotid-Sonde

<220>
<221>  modified_base
<222>  (10)..(10)
<223>  i

<400>  67
cggggttgai cgacaa                                                    16


<210>  68
<211>  16
<212>  DNA
<213>  künstliche Sequenz


<220>
<221>  misc_feature
<222>  (1)..(16)
<223>  Oligonukleotid-Sonde

<400>  68

cggggttgac ccgcaa 16

<210> 69
<211> 16
<212> DNA
<213> künstliche Sequenz

<220>
<221> misc_feature
<222> (1)..(16)
<223> Oligonukleotid-Sonde

<220>
<221> modified_base
<222> (14)..(14)
<223> i

<400> 69
cggggttgac ccgiaa 16

<210> 70
<211> 16
<212> DNA
<213> künstliche Sequenz

<220>
<221> misc_feature
<222> (1)..(16)
<223> Oligonukleotid-Sonde

<220>
<221> modified_base
<222> (11)..(11)
<223> i

<400> 70
cggggttgac icgcaa 16

<210> 71
<211> 15
<212> DNA
<213> künstliche Sequenz

<220>
<221> misc_feature
<222> (1)..(15)
<223> Oligonukleotid-Sonde

<400> 71
cgactgttgg cgctg 15

<210> 72
<211> 15
<212> DNA
<213> künstliche Sequenz

<220>
<221> misc_feature
<222> (1)..(15)
<223> Oligonukleotid-Sonde

<220>
<221>  modified_base
<222>  (10)..(10)
<223>  i

<400>  72
cgactgttgi cgctg                                                        15


<210>  73
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (6)..(6)
<223>  i

<400>  73
cgactittgg cgctg                                                        15


<210>  74
<211>  15
<212>  DNA
<213>  künstliche Sequenz


<220>
<221>  misc_feature
<222>  (1)..(15)
<223>  Oligonukleotid-Sonde

<400>  74
cgactgtggg cgctg                                                        15


<210>  75
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (10)..(10)
<223>  i

<400>  75
cgactgtggi cgctg                                                        15


<210>  76
<211>  15
<212>  DNA

<213>  künstliche Sequenz


<220>
<221>  misc_feature
<222>  (1)..(15)
<223>  Oligonukleotid-Sonde

<220>
<221>  modified_base
<222>  (6)..(6)
<223>  i

<400>  76
cgactitggg cgctg                                                        15


<210>  77
<211>  13
<212>  DNA
<213>  künstliche Sequenz


<220>
<221>  misc_feature
<222>  (1)..(13)
<223>  Oligonukleotid-Sonde

<400>  77
gatcaccacc ggc                                                          13


<210>  78
<211>  13
<212>  DNA
<213>  künstliche Sequenz


<220>
<221>  misc_feature
<222>  (1)..(13)
<223>  Oligonukleotid-Sonde

<220>
<221>  modified_base
<222>  (11)..(11)
<223>  i

<400>  78
gatcaccacc igc                                                          13


<210>  79
<211>  13
<212>  DNA
<213>  künstliche Sequenz


<220>
<221>  misc_feature
<222>  (1)..(13)
<223>  Oligonukleotid-Sonde

<220>
<221>  modified_base
<222>  (7)..(7)
<223>  i

<400>  79
gatcaciacc ggc                                                          13


<210>  80
<211>  14
<212>  DNA
<213>  künstliche Sequenz


<220>
<221>  misc_feature
<222>  (1)..(14)
<223>  Oligonukleotid-Sonde

<400>  80
gatcaccaca ggca                                                         14


<210>  81
<211>  14
<212>  DNA
<213>  künstliche Sequenz


<220>
<221>  misc_feature
<222>  (1)..(14)
<223>  Oligonukleotid-Sonde

<220>
<221>  modified_base
<222>  (12)..(12)
<223>  i

<400>  81
gatcaccaca gica                                                         14


<210>  82
<211>  14
<212>  DNA
<213>  künstliche Sequenz


<220>
<221>  misc_feature
<222>  (1)..(14)
<223>  Oligonukleotid-Sonde

<220>
<221>  modified_base
<222>  (7)..(7)
<223>  i

<400>  82
gatcaciaca ggca                                                         14


<210>  83
<211>  15
<212>  DNA
<213>  künstliche Sequenz

<220>
<221>  misc_feature
<222>  (1)..(15)
<223>  Oligonukleotid-Sonde

<400>  83
gaggcgatag gttgt                                                        15


<210>  84
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (6)..(6)
<223>  i

<400>  84
gaggciatag gttgt                                                        15


<210>  85
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (3)..(3)
<223>  i

<400>  85
gaigcgatag gttgt                                                        15


<210>  86
<211>  15
<212>  DNA
<213>  künstliche Sequenz


<220>
<221>  misc_feature
<222>  (1)..(15)
<223>  Oligonukleotid-Sonde

<400>  86
gagatgatag gttgt                                                        15


<210>  87
<211>  15
<212>  DNA
<213>  künstliche Sequenz


<220>
<221>  misc_feature

<222>  (1)..(15)
<223>  Oligonukleotid-Sonde

<220>
<221>  modified_base
<222>  (6)..(6)
<223>  i

<400>  87
gagatiatag gttgt                                                        15


<210>  88
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (3)..(3)
<223>  i

<400>  88
gaiatgatag gttgt                                                        15


<210>  89
<211>  15
<212>  DNA
<213>  künstliche Sequenz


<220>
<221>  misc_feature
<222>  (1)..(15)
<223>  Oligonukleotid-Sonde

<400>  89
agacgatagg ctgtc                                                        15


<210>  90
<211>  15
<212>  DNA
<213>  künstliche Sequenz


<220>
<221>  misc_feature
<222>  (1)..(15)
<223>  Oligonukleotid-Sonde

<220>
<221>  modified_base
<222>  (13)..(13)
<223>  i

<400>  90
agacgatagg ctitc                                                        15


<210>  91

<211>  15
<212>  DNA
<213>  künstliche Sequenz

<220>
<221>  misc_feature
<222>  (1)..(15)
<223>  Oligonukleotid-Sonde

<220>
<221>  modified_base
<222>  (9)..(9)
<223>  i

<400>  91
agacgataig ctgtc                                                        15

<210>  92
<211>  15
<212>  DNA
<213>  künstliche Sequenz

<220>
<221>  misc_feature
<222>  (1)..(15)
<223>  Oligonukleotid-Sonde
<400>  92
gagacgatag gatgt                                                        15

<210>  93
<211>  15
<212>  DNA
<213>  künstliche Sequenz

<220>
<221>  misc_feature
<222>  (1)..(15)
<223>  Oligonukleotid-Sonde

<220>
<221>  modified_base
<222>  (14)..(14)
<223>  i

<400>  93
gagacgatag gatit                                                        15

<210>  94
<211>  15
<212>  DNA
<213>  künstliche Sequenz

<220>
<221>  misc_feature
<222>  (1)..(15)
<223>  Oligonukleotid-Sonde

<220>
<221>  modified_base
<222>  (10)..(10)

<223>  i

<400>  94
gagacgatai gatgt                                                        15

## Beschreibung

Die vorliegende Erfindung betrifft ein Verfahren zum Nachweis von Mykobakterien-DNA, insbesondere von DNA des Mycobacterium tuberculosis-Komplexes, Oligonukleotide, die als Primer und Sonden in dem Verfahren eingesetzt werden können, einen Träger sowie ein Kit zur Durchführung des Verfahrens.

Tuberkulose (TBC) ist eine durch Mykobakterien verursachte Infektionskrankheit, welche am häufigtsen die Lungen befällt. Der Mycobacterium tuberculosis-Komplex setzt sich zusammen aus M. tuberculosis, M. bovis, M. africanum, M. microti und M. canetti (M. caprae und M. pinnipedii).

Tuberkulose ist weltweit eine der wichtigsten Infektionskrankheiten. Laut Weltgesundheitsorganisation ist ein Drittel der Menschheit mit dem Erreger infiziert, 8 Mio. Menschen erkranken und 1,6 Mio. Menschen (2005) sterben jährlich an Tuberkulose. Damit führt Tuberkulose die weltweite Statistik der tödlichen Infektionserkrankungen an.

Verschärft wird die Bedrohung durch Tuberkulose durch das Auftreten von resistenten Stämmen. Insbesondere das Auftreten sogenannter multiresistenter und/oder extrem resistenter Stämme stellt eine ernsthafte Bedrohung für die Tuberkulosekontrolle dar. Bei den multiresistenten Stämmen handelt es sich um Stämme, welche zumindest gegen die zwei Erstrangmedikamente Isoniazid und Rifampicin resistent sind. Stämme, welche eine extrem resistente Tuberkulose auslösen, sind nicht nur gegen die vorstehend genannten Erstrangmedikamente, sondern noch zumindest gegen ein Fluorochinolon und eines der injizierbaren Zweitrang-Antibiotika Amikacin, Kanamycin und/oder Capreomycin resistent. Mittlerweile konnten sogar schon Stämme beobachtet werden, die Resistenzen gegen alle Antituberkulotika aufwiesen.

Die Therapie von resistenter Tuberkulose ist im Vergleich zur Therapie von sensitiver Tuberkulose (verursacht durch nicht resistente Stämme) schwieriger, da sie im Allgemeinen stärkere Nebenwirkungen hervorruft, die Patienten in der Regel länger infektiös bleiben und die Therapie insgesamt teurer ist.

Im Rahmen der konventionellen Tuberkulose-Diagnostik wird von einem Patienten, bei dem Verdacht auf Tuberkulose besteht, eine Sputum- oder Bronchiallavageprobe entnommen. Danach wird die Probe mit einer Base behandelt bzw. dekontaminiert. Die basisch behandelte bzw. dekontaminierte Probe wird anschließend unter dem Mikroskop auf "säurefeste" Mykobakterien untersucht. Um mikroskopisch die Erkrankung Tuberkulose diagnostizieren zu können, müssen in der untersuchten Probe durchschnittlich 10000 Mykobakterien enthalten sein. Sind weniger Mykobakterien in der Probe enthalten, ist eine Diagnose von Tuberkulose unter dem Mikroskop in der Regel nicht möglich. Dies hat zur Folge, dass insgesamt nur 50% der Tuberkulose positiven Patienten erkannt werden. Der Rest erhält eine Fehldiagnose. Besteht ein dringender Verdacht auf Tuberkulose, werden üblicherweise Kulturen angesetzt, um das Vorliegen von Mykobakterien gegebenenfalls doch noch nachweisen zu können. Nachteilig hierbei ist jedoch, dass der Nachweis von Mykobakterien mittels Kulturen sehr zeitintensiv ist. In der Regel dauert es mehrere Tage bis Wochen, bis diagnostisch verwertbare Ergebnisse vorliegen. In dieser Zeit kann sich eine Tuberkuloseerkrankung ausgehend von den Betroffenen ungehindert ausbreiten.

Der vorliegenden Erfindung liegt daher die Aufgabe zugrunde, geeignete Mittel für einen schnellen und insbesondere sensitiven Nachweis von Tuberkulose-Erregern bzw. Mykobakterien bzw. deren DNA bereitzustellen, welche insbesondere die aus dem Stand der Technik bekannten Unzulänglichkeiten vermeiden und insbesondere auch zum Nachweis von Tuberkulose-Resistenzen geeignet sind.

Diese Aufgabe wird erfindungsgemäß gelöst durch ein in vitro-Verfahren mit den Merkmalen des unabhängigen Anspruchs 1, Oligonukleotid-Primer mit den Merkmalen des unabhängigen Anspruchs 12, Oligonukleotid-Sonden mit den Merkmalen des unabhängigen Anspruchs 13, einen Träger für den Nachweis von Mykobakterien-DNA mit den Merkmalen des unabhängigen Anspruchs 14 sowie durch einen Kit mit den Merkmalen des unabhängigen Anspruchs 15. Bevorzugte Ausführungsformen sind Gegenstand der abhängigen Ansprüche. Der Wortlaut sämtlicher Ansprüche wird hiermit durch Bezugnahme zum Inhalt der Beschreibung gemacht.

Bei dem erfindungsgemäßen Verfahren handelt es sich um ein in-vitro-Verfahren (ex vivo-Verfahren) zum Nachweis von Tuberkulose-Erregern bzw. Mykobakterien bzw. deren DNA, umfassend die folgenden Schritte:
- Bereitstellen einer Probe, enthaltend Mykobakterien bzw. gegebenenfalls enthaltend Mykobakterien,
- Inkubieren der Probe mit einer Base,
- Isolieren von Mykobakterien-DNA aus den Mykobakterien,
- Amplifizieren der isolierten Mykobakterien-DNA, in der Regel mittels Polymerase-Ketten-Reaktion (PCR), wobei vorzugsweise mehr als 30 Amplifikationszyklen durchgeführt werden,
- Nachweisen, insbesondere Identifizieren und vorzugsweise Differenzieren, der amplifizierten Mykobakterien-DNA bzw. der Mykobakterien-DNA-Amplifikate.

Mit anderen Worten betrifft die vorliegende Erfindung ein in vitro-Verfahren (ex vivo-Verfahren) zum Nachweis von Tuberkulose-Erregern bzw. Mykobakterien bzw. deren DNA in einer Probe, enthaltend Mykobakterien bzw. gegebenenfalls enthaltend Mykobakterien, umfassend die folgenden Schritte:
- Inkubieren der Probe mit einer Base,
- Isolieren von Mykobakterien-DNA aus den Mykobakterien,
- Amplifizieren der isolierten Mykobakterien-DNA, in der Regel mittels Polymerase-Ketten-Reaktion (PCR), wobei vorzugsweise mehr als 30 Amplifikationszyklen durchgeführt werden,
- Nachweisen, insbesondere Identifizieren und vorzugsweise Differenzieren, der amplifizierten Mykobakterien-DNA bzw. der Mykobakterien-DNA-Amplifikate.

Überraschenderweise konnte herausgefunden werden, dass eine basische Vorbehandlung (Dekontamination) einer Mykobakterien enthaltenden Probe eine im Vergleich zum Stand der Technik signifikant stärkere Amplifizierung bzw. Vervielfältigung von Mykobakterien-DNA erlaubt. Mit anderen Worten erlaubt die basische Vorbehandlung die Durchführung einer signifikant höheren Anzahl an Amplifikationszyklen. Dadurch wird eine größere Menge an Mykobakterien-DNA generiert, wodurch die Empfindlichkeit bzw. Sensitivität des Verfahrens steigt. So können mit besonderem Vorteil bereits 10-50 Mykobakterien pro Probe ausreichend sein, um einen positiven Nachweis von Mykobakterien bzw. deren DNA führen zu können. Das Risiko von falschpositiven bzw. falschnegativen Ergebnissen lässt sich hierdurch verringern. Ein weiterer Vorteil besteht darin, dass eine aufwendige und insbesondere zeitintensive Kultivierung zum Nachweis von Tuberkulose-Erregern nicht länger erforderlich ist. Insgesamt bietet das Verfahren daher auch den Vorteil einer schnellen Komplettdiagnose. So ist ein Nachweis von Tuberkulose-Erregern bzw. deren DNA innerhalb eines Tages, insbesondere innerhalb weniger Stunden, beispielsweise innerhalb eines Zeitraumes von 1 bis 10 Stunden, bevorzugt 2 bis 8 Stunden, weiter bevorzugt 3 und 6 Stunden, möglich. Abhängig von dem Untersuchungsergebnis kann in kürzester Zeit über eine patientenindividuelle Therapie entschieden werden. Durch die basische Vorbehandlung als solche wird zum Einen schnell wachsende, insbesondere nicht "säurefeste, Flora in den Proben dekontaminiert, in denen Mykobakterien vermutet werden. Die "säurefesten" Mykobakterien überleben diesen Behandlungsschritt, sind allerdings zumindest zeitweise inaktiviert und damit nicht infektiös. Dies wiederum bedeutet ein geringeres Infektionsrisiko für den Anwender, insbesondere Arzt. Zum Anderen bewirkt eine basische Vorbehandlung bei manchen klinischen Materialien, beispielsweise Sputumproben, eine Herabsetzung der Viskosität, woraus sich gewisse Vorteile für das Probenhandling ergeben können.

Bei den Tuberkulose-Erregern handelt es sich vorzugsweise um Mykobakterien, ausgewählt aus der Gruppe bestehend aus Mycobacterium tuberculosis, Mycobacterium bovis, Mycobacterium africanum, Mycobacterium microti, Mycobacterium canetti, Mycobacterium caprae, Mycobacterium pinnipedii und Kombinationen davon. Erfindungsgemäß kann es sich bei den Tuberkulose-Erregern insbesondere um den sogenannten Mycobacterium tuberculosis-Komplex handeln, der sich aus Mycobacterium tuberculosis, Mycobacterium bovis, Mycobacterium africanum, Mycobacterium microti und Mycobacterium canetti (Mycobacterium caprae und Mycobacterium pinnipedii) zusammensetzt.

In einer bevorzugten Ausführungsform des erfindungsgemäßen Verfahrens werden mehr als 35, insbesondere mehr als 40, bevorzugt mehr als 45, weiter bevorzugt mehr als 48, besonders bevorzugt 50 oder mehr, insbesondere mehr als 55, Amplifikationszyklen durchgeführt, um die isolierte Mykobakterien-DNA zu amplifizieren. Erfindungsgemäß ist es jedoch auch denkbar, mehr als 60 Amplifikationszyklen durchzuführen. Besonders bevorzugt werden zwischen 40 und 60, insbesondere 45 und 55, vorzugsweise 48 und 54, Amplifikationszyklen durchgeführt. Wie bereits erwähnt, bewirkt eine höhere Anzahl an Amplifikationszyklen die Herstellung einer größeren Menge an Mykobakterien-DNA, wodurch die Sensitivität des erfindungsgemäßen Verfahrens im Vergleich zu herkömmlichen Nachweisverfahren signifikant gesteigert wird.

Bei der Probe kann es sich prinzipiell um ein beliebiges biologisches Material handeln, welches entweder direkt einem Patienten entnommen oder nach einer Kultivierung bzw. Anreicherung erhalten wurde. So können als Proben beispielsweise Expektorate jeglicher Art, Speichel, Sputum, Rachenabstriche, Bronchiallavagen, Blut, Lymphe, Hautgewebe, Biopsien, insbesondere Bronchialbioptate und/oder Lungenbioptate, Lymphozytenblutkulturmaterialien, Mykobakterienkulturmaterialien, Kolonien, Flüssigkulturen, Bodenproben, Stuhlproben, Urin und dergleichen verwendet werden. Sputumproben sind jedoch besonders bevorzugt.

Grundsätzlich kann die erfindungsgemäß vorgesehene Base in Form einer flüssige Dispersion, Suspension oder Lösung bereitgestellt werden. Erfindungsgemäß kann eine flüssige Dispersion, Suspension bzw. Lösung der Base weitere Bestandteile, insbesondere Supplemente, beispielsweise N-acetyl-L-Cystein, enthalten. Bevorzugt wird zum Inkubieren der Probe eine wässrige Lösung der Base verwendet.

Die erfindungsgemäß vorgesehene Base stellt in einer weiteren Ausführungsform ein Basengemisch dar.

In einer weitergehenden Ausführungsform wird die Probe mit einer anorganischen Base, vorzugsweise mit einer wässrigen Lösung einer anorganischen Base, inkubiert.

Die Base kann aus der Gruppe bestehend aus Natriumhydroxyid, Kaliumhydroxid, Bariumhydroxid, Calciumhydroxid und Mischungen davon ausgewählt sein. Die Verwendung von Natriumhydroxid bzw. einer wässrigen Lösung von Natriumhydroxid (Natronlauge) ist erfindungsgemäß besonders bevorzugt.

In einer weiteren Ausführungsform wird die Probe mit der Base während eines Zeitraumes von 5 bis 60 min, insbesondere 5 bis 30 min, vorzugsweise 10 bis 20 min, inkubiert. In der Regel beträgt die Inkubationszeit ungefähr 15 min. Dadurch kann mit besonderem Vorteil erreicht werden, dass in der Probe gegebenenfalls enthaltene "säurelabile" Erreger weitestgehend zerstört werden.

In einer weiteren Ausführungsform wird die Probe mit der Base in einem Temperaturbereich von 15 bis 35 °C, bevorzugt von 20 bis 30 °C, inkubiert. In der Regel erfolgt die Inkubation bei Raumtemperatur. In diesen Temperaturbereichen kann mit besonderem Vorteil eine Inaktivierung der Mykobakterien erreicht und gleichzeitig eine Denaturierung ihrer DNA vermieden werden.

Die Inkubation wird in der Regel in hierfür geeigneten Inkubationsgefäßen durchgeführt. Als Inkubationsgefäße kommen beispielsweise Inkubationswannen, Mikrotiterplatten, Lochplatten, Eppendorf-Caps und dergleichen in Betracht. Besonders vorteilhaft ist es, wenn die Probe während des Inkubierens geschüttelt wird. Dadurch kann ein möglichst gleichmäßiges Inkubieren der Probe mit der Base erreicht werden.

Erfindungsgemäß kann es weiterhin vorgesehen sein, die Base nach dem Inkubationsschritt zu entfernen, bevor die DNA aus den Mykobakterien isoliert wird. Hierzu kann die Probe einem oder gegebenenfalls mehreren Waschschritten unterworfen werden. Alternativ oder in Kombination dazu kann die Base aus der Probe mittels eines geeigneten Extraktionsmittels extrahiert werden.

Zur Isolierung der Mykobakterien-DNA wird diese üblicherweise aus den Mykobakterien extrahiert. Das Extrahieren umfasst in aller Regel einen zellulären Aufschluss der Mykobakterien und vorzugsweise eine Vor- bzw. Aufreinigung der mittels des Aufschlusses freigesetzten Mykobakterien-DNA. Zum Aufschluss der Mykobakterien kommen grundsätzlich sowohl mechanische als auch nicht mechanische Aufschlussverfahren in Betracht. Beispielsweise können die Mykobakterien in kleinen Mengen im Mörser mit Sand oder Al₂O₃ oder in einer Glasperlenmühle zerrieben werden. Eine weitere Möglichkeit, Mykobakterien aufzuschließen, besteht in einer Ultraschallbehandlung der Zellen. Hierbei werden die Mykobakterien durch ständiges Aneinanderstoßen zerstört (Kavitationskräfte). Eine weitere Möglichkeit, die Mykobakterien aufzuschließen, besteht darin, dass man in einem ersten Schritt durch Behandlung mit Lysozym die Peptidoglykanhülle und in einem zweiten Schritt durch Behandlung mit einem nicht ionischen Tensid wie beispielsweise Triton X-100 (C₁₄H₂₂O(C₂H₄O)ₙ) die Zellmembran der Bakterien zerstört. Zur Vor- bzw. Aufreinigung der Mykobakterien-DNA kommen grundsätzlich alle dem Fachmann geläufigen Reinigungstechniken in Betracht, beispielsweise Ionenaustauscherchromatographie, Gel-Permeations-Chromatographie (GPC), Affinitätschromatographie oder Gelelektrophorese.

In der Regel wird die Mykobakterien-DNA mittels geeigneter Primer amplifiziert. Bei den Primern handelt es sich um einzelsträngige Oligonukleotidsequenzen, welche als Startpunkt für die Synthese eines Primer-Verlängerungsproduktes, das dem zu kopierenden Nukleinsäurestrang komplementär ist, fungieren kann. Die Länge sowie die Sequenz der Primer sind zweckmäßigerweise derart gewählt, dass sie eine im Folgenden noch näher beschriebene Primer-Hybridisierung ermöglichen. Die Primer sind vorzugsweise etwa 5 bis 50 Nukleotide lang. Die spezifische Länge und Sequenz der Primer hängt dabei insbesondere von der Komplexität der benötigten Mykobakterien-DNA ab, ebenso wie von den Bedingungen der Primerverwendung wie beispielsweise Temperatur und Ionenstärke. Vorzugsweise werden zum Amplifizieren der Mykobakterien-DNA Primer, ausgewählt aus der Gruppe bestehend aus Oligonukleotide gemäß den SEQ ID NO 1 bis 10 der Tabelle 1 bzw. des beiliegenden Sequenzprotokolls und Kombinationen davon, eingesetzt. Die Primer können insbesondere Oligonukleotidsequenzen, ausgewählt aus der Gruppe bestehend aus Oligonukleotide gemäß den SEQ ID NO 1 bis 10 der Tabelle 1 bzw. des beiliegenden Sequenzprotokolls und Kombinationen davon, umfassen.

Wie bereits erwähnt, wird die isolierte Mykobakterien-DNA in der Regel mittels einer Polymerase-Ketten-Reaktion (PCR) amplifiziert bzw. vervielfältigt. Hierbei wird in einem ersten Schritt, der auch als Denaturierungsschritt bezeichnet wird, die doppelsträngige Mykobakterien-DNA auf 94 bis 96 °C erhitzt, um die Stränge zu trennen. Die Trennung der DNA-Stränge beruht darauf, dass die Wasserstoffbrückenbindungen, die die beiden DNA-Stränge zusammenhalten, aufgebrochen werden. Im ersten Amplifikationszyklus wird die Mykobakterien-DNA häufig für längere Zeit erhitzt (Initialisierung), um sicherzustellen, dass sich sowohl die Ausgangs-DNA als auch die zur Amplifikation verwendeten Primer vollständig voneinander getrennt haben und nur noch Einzelstränge vorliegen. Werden sogenannte Hot-Start-Polymerasen verwendet, müssen diese in der Regel durch eine noch längere anfängliche Erhitzungsphase (bis zu 15 Minuten) aktiviert werden. In einem zweiten Schritt, der sogenannten Primer-Hybridisierung, wird die Temperatur in dem Thermocycler, in der Regel zumindest ca. 30 Sekunden lang, auf eine Temperatur gehalten, die eine spezifische Anlagerung der Primer an die DNA erlaubt. Die genaue Temperatur wird hierbei durch die Länge und die Sequenz der Primer bestimmt. Wird die Temperatur zu niedrig gewählt, können sich die Primer unter Umständen auch an nicht 100%-komplementäre Sequenzen unter Ausbildung von unspezifischen Produkten ("Geisterbanden") anlagern. Wird die Temperatur zu hoch gewählt, ist die thermische Bewegung der Primer unter Umständen so groß, dass sie sich nicht richtig anheften können, so dass es zu gar keiner oder nur zu einer ineffizienten Produktbildung kommt. Die Temperatur, welche die beiden vorstehend beschriebenen Effekte weitgehend ausschließt, liegt normalerweise 2 bis 3 °C unter dem Schmelzpunkt der Primersequenzen. Dies entspricht gewöhnlich einer Temperatur zwischen 55 und 65 °C. In einem dritten Schritt, der sogenannten Elongation, füllt eine DNA-Polymerase die fehlenden Stränge mit freien Nukleotiden auf. Sie beginnt dabei am 3'-Ende des angelagerten Primers und folgt dann dem DNA-Strang. Der Primer wird nicht wieder abgelöst, er bildet vielmehr den Anfang des neuen Einzelstrangs. Dieser Schritt dauert gewöhnlich etwa 30 Sekunden je 500 Basenpaare, variiert aber in Abhängigkeit von der verwendeten DNA-Polymerase. Die Temperatur hängt vom Arbeitsoptimum der verwendeten DNA-Polymerase ab und kann beispielsweise zwischen 68 und 72 °C liegen. Besonders vorteilhaft ist die Verwendung von sogenannten thermostabilen DNA-Polymerasen, d.h. Enzymen, die auch bei Temperaturen zwischen 90°C und annähernd 100°C ihre Polymerase-Aktivität behalten.

In einer weiterführenden Ausführungsform werden zum Amplifizieren der Mykobakterien-DNA Primer eingesetzt, die mit einer den Nachweis der Amplifikate unterstützenden Verbindung, beispielsweise Biotin, konjugiert sind. Die mittels derartiger Primer hergestellten DNA-Amplifikate sind bei dieser Ausführungsform ebenfalls mit der Verbindung konjugiert. Werden beispielsweise biotinylierte Primer verwendet, so liegen auch die damit hergestellten DNA-Amplifikate biotinyliert vor. Biotinylierte DNA-Amplifikate können an eine Streptavidin gekoppelte Verbindung, beispielsweise Streptavidin gekoppelte alkalische Phosphatase, binden. Diese Bindung kann für einen kolorimetrischen Nachweis der DNA-Amplifikate verwendet werden, worauf im Folgenden noch näher eingegangen wird.

Bevorzugt werden die Mykobakterien-DNA-Amplifikate mittels Hybridisierung, in der Regel mittels reverser Hybridisierung, mit Oligonukleotid-Sonden (Gensonden) nachgewiesen. Unter Oligonukleotid-Sonden im Sinne der vorliegenden Erfindung werden sequenzspezifische Einzelstrang-Oligonukleotide verstanden, deren Nukleotidsequenzen komplementär bzw. homolog zu Nukleotidsequenzen bzw. Nukleotidsequenzabschnitten von Mykobakterien-DNA-Amplifikaten sind. Unter dem Begriff komplementär, wie er in der vorliegenden Beschreibung verwendet wird, soll verstanden werden, dass die Sequenz eines Einzelstrang-Oligonukleotids zu der Sequenz einer einzelsträngigen Mykobakterien-DNA komplementär, vorzugsweise vollständig komplementär, ist.

Bevorzugt sind die Oligonukleotid-Sonden komplementär zu Sequenzen bzw. Sequenzabschniten von Aktivierungs- und/oder Resistenzgenen für Antituberkulotika, insbesondere Antibiotika. Dadurch lassen sich sensible Mykobakterien und/oder resistente, insbesondere multiresistente und/oder extrem resistente, Mykobakterien nachweisen. Insbesondere ist eine Differenzierung zwischen sensiblen und resistenten, insbesondere multiresistenten und/oder extrem resistenten, Mykobakterien möglich. Unter sensiblen Mykobakterien im Sinne der vorliegenden Erfindung sollen Mykobakterien verstanden werden, welche auf ein Antituberkulotikum bzw. auf eine Kombination von Antituberkulotika ansprechen.

Bevorzugt werden degenerierte, insbesondere inosinhaltige bzw. Inosin umfassende, Oligonukleotid-Sonden zum Nachweis der amplifizierten Mykobakterien-DNA eingesetzt. Die Verwendung von Inosin hat den Vorteil, dass es in einer Wobble-Position mit allen vier DNA-Basen Adenin (A), Guanin (G), Cytosin (C) und Thymin (T) Basenpaarungen eingeht. Mit anderen Worten kann Inosin alle 4 DNA-Basen ersetzen. Allerdings zeigt Inosin ein im Vergleich zu den anderen Basen schwächeres Bindungsverhalten. Dies kann jedoch in besonders vorteilhafter Weise bei der Mutationsanalyse ausgenutzt werden. Bevorzugt wird hierzu bei einer Oligonukleotid-Sonde eine Base, welche drei Wasserstoffbindungen eingehen kann (G,C), in der Nähe einer Mutation durch Inosin ersetzt. Durch das schwächere Bindungsverhalten in der Nähe der Mutation wird das Bindungsverhalten der Basen, welche in der Mutationsregion liegen, vergleichsweise aufgewertet. Dies erhöht die Spezifität der Oligonukleotid-Sonde bzw. senkt das Risiko von falschpositiven Bindungen mit einer Wildtyp-Mykobakterien-DNA.

Bei den Oligonukleotid-Sonden kann es sich in einer weiteren Ausführungsform um sogenannte Wildtyp-Sonden und/oder Mutations-Sonden handeln. Unter Wildtypsonden im Sinne der vorliegenden Erfindung sollen Oligonukleotid-Sonden verstanden werden, welche komplementär bzw. homolog zu einem Antituberkulotikum-Aktivierungsgen bzw. einem Abschnitt davon, insbesondere einem Antibiotikum-Aktivierungsgen bzw. einem Abschnitt davon, auf der Mykobakterien-DNA sind. Unter Mutations-Sonden im Sinne der vorliegenden Erfindung sollen Oligonukleotide-Sonden verstanden werden, welche komplementär bzw. homolog zu einem Antituberkulotikum-Resistenzgen bzw. einem Abschnitt davon, insbesondere einem Antibiotikum-Resistenzgen bzw. einem Abschnitt davon, auf der Mykobakterien-DNA sind.

Geeignete Hybridisierungsverfahren zum Nachweis der Mykobakterien-DNA-Amplifikate stellen beispielsweise Dot-Plot-Verfahren, Southern-Plot-Verfahren, Sandwich-Verfahren und dergleichen dar. Erfindungsgemäß ist es besonders bevorzugt, wenn die amplifizierte Mykobakterien-DNA mittels reverser Hybridisierung nachgewiesen wird.

Die Oligonukleotid-Sonden sind vorzugsweise auf einem geeigneten Träger, beispielsweise auf einem Nitrocellulosestreifen, immobilisiert. Zur Immobilisierung können die Oligonukleotid-Sonden an ihrem 3'-Ende jeweils mit einem Oligo(T)-Schwanz oder mit einem Poly(T)-Schwanz versehen sein.

Bevorzugt werden die Mykobakterien-DNA-Amplifikate auf einen Träger, auf dessen Oberfläche sequenzspezifische Oligonukleotid-Sonden immobilisiert sind, aufgebracht. Mit besonderem Vorteil wird ein Hybridisierungspuffer zugesetzt, um die Hybridsierung zwischen den DNA-Amplifikaten und den hierzu komplementären Oligonukleotidsequenzen zu erleichtern bzw. zu beschleunigen. Um sicherzustellen, dass nur Hybride nachgewiesen werden, die aus DNA-Amplifikaten und hierzu komplementären, vorzugsweise vollständig komplementären, Oligonukleotid-Sonden bestehen, können mehrere hochspezifische Waschschritte vorgesehen sein.

Der Nachweis einer erfolgreichen Hybridisierung zwischen Mykobakterien-DNA-Amplifikaten und hierzu komplementären Oligonukleotid-Sonden kann mittels radioaktiver Markierungen oder geeigneter Farbreaktionen geführt werden. Sind die Mykobakterien-DNA-Amplifikate beispielsweise mit Biotin konjugiert, kann eine erfolgreiche Hybridisierung mit Streptavidin gekoppelter alkalischer Phosphatase und geeigneten chromogenen Substraten für das Enzym, beispielsweise NBT (Nitroblau-Tetrazoliumchlorid) und BCIP (5-Brom-4-Chlor-3-Indoxyl-phosphat), nachgewiesen werden.

Vorzugsweise wird die amplifizierte Mykobakterien-DNA mittels Oligonukleotid-Sonden, ausgewählt aus der Gruppe bestehend aus Oligonukleotide gemäß den SEQ ID NO 11 bis 94 der Tabelle 2 bzw. des beiliegenden Sequenzprotokolls und Kombinationen davon, nachgewiesen. In einer weiteren Ausführungsform werden Oligonukleotid-Sonden verwendet, die Oligonukleotide gemäß den SEQ ID NO 11 bis 94 der Tabelle 2 bzw. des beiliegenden Sequenzprotokolls und Kombinationen davon umfassen.

In einer bevorzugten Ausführungsform wird das Verfahren zum Nachweis einer Tuberkulose-Infektion, einer Tuberkuloseerkrankung und/oder von Tuberkuloseresistenzen, insbesondere Multiresistenzen und/oder Extremresistenzen, verwendet. Insbesondere können mit Hilfe des Verfahrens in der Mykobakterien-DNA enthaltene Aktivierungs- und/oder Resistenzgene für Antituberkulotika, insbesondere Antibiotika, nachgewiesen werden. Bei den nachzuweisenden Genen kann es sich insbesondere um Aktivierungs- und/oder Resistenzgene für Isoniazid, Rifampicin, Rifabutin, Chinolone, insbesondere Fluorchinolone, Amikacin, Kanamycin, Capreomycin, Streptomycin, Aminoglykoside und Kombinationen davon handeln. Die Ausbildung von Resistenzen kann beispielsweise auf Mutationen von Aktivierungsgenen beruhen, wodurch die ihre gegenüber Antituberkulotika aktivierende Wirkung eingebüßt haben. Mit anderen Worten kann es sich bei den Resistenzgenen um mutierte bzw. Mutationen aufweisende Aktivierungsgene handeln. Das Antituberkulotikum Isoniazid wird beispielsweise durch die bakterielle Katalase-Peroxidase aktiviert. Isoniazid selbst zeigt nur gegenüber Mykobakterien eine bakterizide Wirkung. Im aktivierten Zustand hemmt Isoniazid die Synthese von Mykolsäure, die ein essentieller Bestandteil der mykobakteriellen Zellwand darstellt. Die hemmende Wirkung beruht auf einer Bindung von Isoniazid an Enoylreduktase, die wiederum einen frühen Schritt der Fettsäuresynthese katalysiert. Resistenzen gegenüber Isoniazid sind vor allem auf chromosomale Mutationen des bakterielle Katalase-Peroxidase-Gens (katG) zurückzuführen. Da dieses Gen das einzige Enzym (Katalase-Peroxidase) exprimiert, das Isoniazid aktivieren kann, führen Mutationen zwangsläufig zur Entstehung von Resistenzen. Auch eine Überexpression der Enoylreduktase, die auf eine chromosomale Mutation der Promotorregion des Enoylreduktase-Gens (InhA) zurückzuführen sein kann, kann Ursache für eine Isoniazid-Resistenz sein. Erfindungsgemäß kann das Verfahren daher insbesondere zur Diskriminierung zwischen sensiblen Tuberkulose-Erregern bzw. Mykobakterien und resistenten, insbesondere multiresistenten und/oder extrem resistenten, Tuberkulose-Erregern bzw. Mykobakterien, verwendet werden. Die wichtigsten Mutationen liegen bei dem bakterielle Katalase-Peroxidase-Gen auf dem Codon 315 (katG 315) und bei dem Enoylreduktase-Gen auf den Codons -8, -15 und -16 (InhA 8, InhA 15 und InhA 16). Das Antituberkulotikum Rifampicin hemmt die bakterielle RNA-Polymerase, indem es an deren beta-Untereinheit bindet, wodurch die Transkription der RNA-Polymerase verhindert wird, was schließlich zum Zelltod führt. Resistenzen beruhen gewöhnlich auf chromosomalen Mutationen im rpoB-Gen, welches die beta-Untereinheit der RNA-Polymerase codiert. Diese chromosomalen Mutationen führen zu Veränderungen der Bindungsstelle und einer geringeren Bindungsaffinität von Rifampicin. Die wichtigsten Mutationen liegen auf den Codons 516, 526 und 531 (rpoB 516, rpoB 526 und rpoB 531).

In einer weitergehenden Ausführungsform lassen sich Genmutationen der Mykobakterien-DNA, ausgewählt aus der Gruppe bestehend aus katG-Genmutationen, inhA-Genmutationen, rpoB-Genmutationen und Kombinationen davon, nachweisen.

Eine bevorzugte katG-Genmutation, welche mittels des erfindungsgemäßen Verfahrens nachgewiesen werden kann, ist S315T, eine Mutation in Codon 315 des katG-Gens darstellend, die zu einem Austausch der Aminosäure S gegen T führt.

Besonders bevorzugte inhA-Genmutationen sind ausgewählt aus der Gruppe bestehend aus:
- A16G, eine Mutation in Codon 16 des inhA-Gens darstellend, die zu einem Austausch der Aminosäure A gegen G führt,
- C15T eine Mutation in Codon 15 des inhA-Gens darstellend, die zu einem Austausch der Aminosäure C gegen T führt;
- T8A eine Mutation in Codon 8 des inhA-Gens darstellend, die zu einem Austausch der Aminosäure T gegen A führt,
- T8C eine Mutation in Codon 8 des inhA-Gens darstellend, die zu einem Austausch der Aminosäure T gegen C führt und Kombinationen davon.

Besonders bevorzugte Mutationen des rpoB-Gens sind aus der Gruppe ausgewählt aus der Gruppe bestehend aus:
- D516V eine Mutation in Codon 516 des rpoB-Gens darstellend, die zu einem Austausch der Aminosäure D gegen V führt,
- D516Y eine Mutation in Codon 516 des rpoB-Gens darstellend, die zu einem Austausch der Aminosäure D gegen Y führt,
- H526Y eine Mutation in Codon 526 des rpoB-Gens darstellend, die zu einem Austausch der Aminosäure H gegen Y führt,
- H526D eine Mutation in Codon 526 des rpoB-Gens darstellend, die zu einem Austausch der Aminosäure H gegen D führt,
- H526R eine Mutation in Codon 526 des rpoB-Gens darstellend, die zu einem Austausch der Aminosäure H gegen R führt,
- S531 L eine Mutation in Codon 531 des rpoB-Gens darstellend, die zu einem Austausch der Aminosäure S gegen L führt,
- S531W eine Mutation in Codon 531 des rpoB-Gens darstellend, die zu einem Austausch der Aminosäure S gegen W führt und Kombinationen davon.

Erfindungsgemäß ist der Nachweis einer Isoniazid-Resistenz und/oder Rifampicin-Resistenz besonders bevorzugt.

Von der vorliegenden Erfindung werden auch Oligonukleotid-Primer zur Herstellung von Mykobakterien-DNA-Amplifikaten erfasst. Die Oligonukleotid-Primer sind ausgewählt aus der Gruppe bestehend aus Oligonukleotide gemäß den SEQ ID NO 1 bis 10 der Tabelle 1 bzw. des beiliegenden Sequenzprotokolls und Kombinationen davon. Bezüglich weiterer Merkmale und Vorteile wird vollständig auf die bisherige und die noch folgende Beschreibung Bezug genommen.

Ein weiterer Gegenstand der vorliegenden Erfindung betrifft Oligonukleotid-Sonden, insbesondere zum Nachweis, insbesondere zum Identifizieren und vorzugsweise Differenzieren, von Mykobakterien-DNA-Amplifikaten. Die Oligonukleotid-Sonden sind ausgewählt aus der Gruppe bestehend aus Oligonukleotide gemäß den SEQ ID NO 11 bis 94 der Tabelle 2 bzw. des beiliegenden Sequenzprotokolls und Kombinationen davon. Bezüglich weiterer Merkmale und Vorteile wird ebenfalls vollständig auf die bisherige und die noch folgende Beschreibung Bezug genommen.

Weiterhin umfasst die Erfindung einen Träger zum Nachweisen von Mykobakterien-DNA-Amplifikaten, umfassend Oligonukleotide, ausgewählt aus der Gruppe bestehend aus Oligonukleotide gemäß den SEQ ID NO 11 bis 94 der Tabelle 2 bzw. des beiliegenden Sequenzprotokolls und Kombinationen davon. Bei dem Träger kann es sich im Prinzip um ein beliebiges Substrat handeln, an das eine oder mehrere Oligonukleotid-Sonden gekoppelt werden kann, vorausgesetzt, dass die Oligonukleotid-Sonde bzw. -Sonden ihre Hybridisierungseigenschaften behalten, und vorausgesetzt, dass das Hintergrundniveau der Hybridisierung niedrig bleibt. Bevorzugt handelt es sich bei dem Träger um eine Mikrotiterplatte, Lochplatte, eine Membran (beispielsweise Nylon oder Nitrocellulose) oder ein Mikrokügelchen (Beat). Vor dem Auftragen der Oligonukleotid-Sonden auf den Träger oder zur Immobilisierung bzw. Fixierung auf diesem kann es zweckmäßig sein, die Oligonukleotid-Sonden zu modifizieren, um die Immobilisierung und/oder die Hybridisierungseffizienz zu steigern. Der Träger ist bevorzugt ein Teststreifen, insbesondere ein Nitrocellulose-Teststreifen. Solche Modifikationen können beispielsweise ein Homopolymer-Tailing, eine Kopplung mit unterschiedlichen reaktiven Gruppen, beispielsweise aliphatischen Gruppen, Amino-Gruppen, Schwefelwasserstoff-Gruppen, Carboxyl-Gruppen und/oder eine Kopplung mit Biotin, Haptenen oder Proteinen beinhalten. Zur Immobilisierung auf der Oberfläche des Trägers sind die Oligonukleotide an ihrem 3'-Ende vorzugsweise mit einem Oligo(T)-Schwanz oder einem Poly(T)-Schwanz versehen sein. Bezüglich weiterer Merkmale und Vorteile wird ebenso vollständig auf die bisherige und die noch folgende Beschreibung Bezug genommen.

Weiterhin ist Gegenstand der vorliegenden Erfindung auch ein Kit, insbesondere zur Verwendung in dem erfindungsgemäßen Verfahren, zumindest umfassend eine der folgenden Komponenten:
- Oligonukleotid-Primer, ausgewählt aus der Gruppe bestehend aus Oligonukleotide gemäß der SEQ ID NO 1 bis 10 der Tabelle 1 bzw. des beiliegenden Sequenzprotokolls und Kombinationen davon und/oder
- Oligonukleotid-Sonden, ausgewählt aus der Gruppe bestehend aus Oligonukleotide gemäß der SEQ ID NO 11 bis 94 der Tabelle 2 bzw. des beiliegenden Sequenzprotokolls und Kombinationen davon.

Das erfindungsgemäße Kit eignet sich demnach insbesondere zum Nachweis von Tuberkulose-Erregern bzw. Mykobakterien bzw. deren DNA in einer Probe. In einer weitergehenden Ausführungsform eignet sich das Kit zum Nachweis bzw. zur Diagnose einer Tuberkulose-Infektion oder einer Tuberkuloseerkrankung. Bevorzugt wird das erfindungsgemäße Kit zum Nachweis von Antituberkulotika-Resistenzen, insbesondere einer Isonyacid-Resistenz und/oder Rifampicin-Resistenz, verwendet. Bezüglich weiterer Antituberkulotika-Resistenzen, welche sich grundsätzlich mittels des erfindungsgemäßen Kits nachweisen lassen, wird vollständig auf die bisherige Beschreibung Bezug genommen.

In einer weiteren Ausführungsform weist das Kit eine zusätzliche Komponente auf, welche ausgewählt ist aus der Gruppe bestehend aus Denaturierungsreagens, Hybridisierungspuffer, Waschlösung bzw. Waschpuffer, DNA-Polymerase, Polymerasepuffer, Träger für Oligonucleotid-Sonden, Träger mit Oligonucleotid-Sonden, Enzym-Konjugat, insbesondere Streptavidin gekoppelte alkalische Phosphatase, Konjugat-Puffer, Farbstoffe wie beispielsweise NBT und/oder BCIP, Inkubationsbehältnis, insbesondere Inkubationswanne, und Kombinationen davon.

Unter dem Begriff "Waschlösung" soll im Sinne der vorliegenden Erfindung eine Lösung verstanden werden, welche das Waschen von gebildeten Hybriden aus einzelsträngiger Mykobakterien-DNA und einzelsträngigen Oligonukleotid-Sonden unter den entsprechenden Stringenzbedingungen ermöglicht. Unter dem Begriff "Hybridisierungspuffer" soll im Sinne der vorliegenden Erfindung ein Puffer verstanden werden, der zwischen den Oligonukleotid-Sonden und den Mykobakterien-DNA-Amplifikaten eine unter den entsprechenden Stringenzbedingungen auftretende Hybridisierungsreaktion ermöglicht.

Bezüglich weiterer Merkmale und Vorteile zu dem Kit wird ebenso vollständig auf die bisherige und die noch folgende Beschreibung Bezug genommen.

Die in der vorliegenden Beschreibung gewählte Formulierung "... ausgewählt aus der Gruppe bestehend aus Oligonukleotide gemäß den SEQ ID NO 1 bis 10 der Tabelle 1 bzw. des beiliegenden Sequenzprotokolls und Kombinationen davon" ist gleichbedeutend mit der Formulierung "...ausgewählt aus der Gruppe bestehend aus Oligonukleotid gemäß SEQ ID NO 1, Oligonukleotid gemäß SEQ ID NO 2, Oligonukleotid gemäß SEQ ID NO 3, Oligonukleotid gemäß SEQ ID NO 4, Oligonukleotid gemäß SEQ ID NO 5, Oligonukleotid gemäß SEQ ID NO 6, Oligonukleotid gemäß SEQ ID NO 7, Oligonukleotid gemäß SEQ ID NO 8, Oligonukleotid gemäß SEQ ID NO 9, Oligonukleotid gemäß SEQ ID NO 10 der untenstehenden Tabelle 1 bzw. des beiliegenden Sequenzprotokolls und Kombinationen davon".

**Tabelle 1: Beispiele für erfindungsgemäß bevorzugte Oligonukleotid-Primer.**

| Oligonukleotid-Primer | Zielregion | Sequenz |
|---|---|---|
| SEQ ID NO 1 | gyrB | caaatcgtttgtgcagaagg |
| SEQ ID NO 2 | gyrB | cigaacttcggtgttctta gc |
| SEQ ID NO 3 | katG | atgaacgacgtcgaaacagc |
| SEQ ID NO 4 | katG | cgtagccgtacaggatctcg |
| SEQ ID NO 5 | inhA | agaaagggatccgtcatgg |
| SEQ ID NO 6 | inhA | actgaacgggatacgaatgg |
| SEQ ID NO 7 | rpoB | atgaccacccaggacgtg |
| SEQ ID NO 8 | rpoB | gcacgctcacgtgacagac |
| SEQ ID NO 9 | | gaaggtgaaggtcggagtc |
| SEQ ID NO 10 | | gaagatggtgatgggatttc |

Die in der vorliegenden Beschreibung gewählte Formulierung "... ausgewählt aus der Gruppe bestehend aus Oligonukleotide gemäß den SEQ ID NO 11 bis 94 der Tabelle 2 bzw. des beiliegenden Sequenzprotokolls und Kombinationen davon" ist gleichbedeutend mit der Formulierung "...ausgewählt aus der Gruppe bestehend aus Oligonukleotid gemäß SEQ ID NO 11, Oligonukleotid gemäß SEQ ID NO 12, Oligonukleotid gemäß SEQ ID NO 13, Oligonukleotid gemäß SEQ ID NO 14, Oligonukleotid gemäß SEQ ID NO 15, Oligonukleotid gemäß SEQ ID NO 16, Oligonukleotid gemäß SEQ ID NO 17, Oligonukleotid gemäß SEQ ID NO 18, Oligonukleotid gemäß SEQ ID NO 19, Oligonukleotid gemäß SEQ ID NO 20, Oligonukleotid gemäß SEQ ID NO 21, Oligonukleotid gemäß SEQ ID NO 22, Oligonukleotid gemäß SEQ ID NO 23, Oligonukleotid gemäß SEQ ID NO 24, Oligonukleotid gemäß SEQ ID NO 25, Oligonukleotid gemäß SEQ ID NO 26, Oligonukleotid gemäß SEQ ID NO 27, Oligonukleotid gemäß SEQ ID NO 28, Oligonukleotid gemäß SEQ ID NO 29, Oligonukleotid gemäß SEQ ID NO 30, Oligonukleotid gemäß SEQ ID NO 31, Oligonukleotid gemäß SEQ ID NO 32, Oligonukleotid gemäß SEQ ID NO 33, Oligonukleotid gemäß SEQ ID NO 34, Oligonukleotid gemäß SEQ ID NO 35, Oligonukleotid gemäß SEQ ID NO 36, Oligonukleotid gemäß SEQ ID NO 37, Oligonukleotid gemäß SEQ ID NO 38, Oligonukleotid gemäß SEQ ID NO 39, Oligonukleotid gemäß SEQ ID NO 40, Oligonukleotid gemäß SEQ ID NO 41, Oligonukleotid gemäß SEQ ID NO 42, Oligonukleotid gemäß SEQ ID NO 43, Oligonukleotid gemäß SEQ ID NO 44, Oligonukleotid gemäß SEQ ID NO 45, Oligonukleotid gemäß SEQ ID NO 46, Oligonukleotid gemäß SEQ ID NO 47, Oligonukleotid gemäß SEQ ID NO 48, Oligonukleotid gemäß SEQ ID NO 49, Oligonukleotid gemäß SEQ ID NO 50, Oligonukleotid gemäß SEQ ID NO 51, Oligonukleotid gemäß SEQ ID NO 52, Oligonukleotid gemäß SEQ ID NO 53, Oligonukleotid gemäß SEQ ID NO 54, Oligonukleotid gemäß SEQ ID NO 55, Oligonukleotid gemäß SEQ ID NO 56, Oligonukleotid gemäß SEQ ID NO 57, Oligonukleotid gemäß SEQ ID NO 58, Oligonukleotid gemäß SEQ ID NO 59, Oligonukleotid gemäß SEQ ID NO 60, Oligonukleotid gemäß SEQ ID NO 61, Oligonukleotid gemäß SEQ ID NO 62, Oligonukleotid gemäß SEQ ID NO 63, Oligonukleotid gemäß SEQ ID NO 64, Oligonukleotid gemäß SEQ ID NO 65, Oligonukleotid gemäß SEQ ID NO 66, Oligonukleotid gemäß SEQ ID NO 67, Oligonukleotid gemäß SEQ ID NO 68, Oligonukleotid gemäß SEQ ID NO 69, Oligonukleotid gemäß SEQ ID NO 70, Oligonukleotid gemäß SEQ ID NO 71, Oligonukleotid gemäß SEQ ID NO 72, Oligonukleotid gemäß SEQ ID NO 73, Oligonukleotid gemäß SEQ ID NO 74, Oligonukleotid gemäß SEQ ID NO 75, Oligonukleotid gemäß SEQ ID NO 76, Oligonukleotid gemäß SEQ ID NO 77, Oligonukleotid gemäß SEQ ID NO 78, Oligonukleotid gemäß SEQ ID NO 79, Oligonukleotid gemäß SEQ ID NO 80, Oligonukleotid gemäß SEQ ID NO 81, Oligonukleotid gemäß SEQ ID NO 82, Oligonukleotid gemäß SEQ ID NO 83, Oligonukleotid gemäß SEQ ID NO 84, Oligonukleotid gemäß SEQ ID NO 85, Oligonukleotid gemäß SEQ ID NO 86, Oligonukleotid gemäß SEQ ID NO 87, Oligonukleotid gemäß SEQ ID NO 88, Oligonukleotid gemäß SEQ ID NO 89, Oligonukleotid gemäß SEQ ID NO 90, Oligonukleotid gemäß SEQ ID NO 91, Oligonukleotid gemäß SEQ ID NO 92, Oligonukleotid gemäß SEQ ID NO 93, Oligonukleotid gemäß SEQ ID NO 94 der untenstehenden Tabelle 2 bzw. des beiliegenden Sequenzprotokolls und Kombinationen davon".

**Tabelle 2: Beispiele für erfindungsgemäß bevorzugte Oligonukleotid-Sonden. In der Tabelle 2 steht der Kleinbuchstabe "i" für Inosin (wie auch im beiliegenden Sequenzprotokoll).**

| Oligonukleotid-Sonde | Zielregion | Sequenz |
|---|---|---|
| SEQ ID NO 11 | gyrB | cgaactgtatgtcgtag |
| SEQ ID NO 12 | gyrB | cgaactitatgtcgtag |
| SEQ ID NO 13 | gyrB | cgaactgtatitcgtag |
| SEQ ID NO 14 | inhA | gagacgataggttgt |
| SEQ ID NO 15 | inhA | agatacgataggttgt |
| SEQ ID NO 16 | inhA | gagacitataggttgt |
| SEQ ID NO 17 | katG | gatcaccagcggc |
| SEQ ID NO 18 | katG | galcaccagclgc |
| SEQ ID NO 19 | katG | gatcaclagcggc |
| SEQ ID NO 20 | rpoB | ccaattcatggacc |
| SEQ ID NO 21 | rpoB | ccaaltcalggact |
| SEQ ID NO 22 | rpoB | ccaattcatlgacc |
| SEQ ID NO 23 | rpoB | cggggttgacccacaa |
| SEQ ID NO 24 | rpoB | cggggttgalccacaa |
| SEQ ID NO 25 | rpoB | cggggltgacccataa |
| SEQ ID NO 26 | rpoB | cgactgtcggcgctg |
| SEQ ID NO 27 | rpoB | cgactltcggcgctg |
| SEQ ID NO 28 | rpoB | gactgtcglcgctg |
| SEQ ID NO 29 | rrs | gcagccgcggtaata |
| SEQ ID NO 30 | rrs | gcagctgcggtaata |
| SEQ ID NO 31 | rrs | gcagccgclgtaata |
| SEQ ID NO 32 | rpsL | caccactccgaagaag |
| SEQ ID NO 33 | rpsL | caccaclccgaataag |
| SEQ ID NO 34 | rpsL | caccactcclaagaag |
| SEQ ID NO 35 | rpsL | ccactccgaagaagcc |
| SEQ ID NO 36 | rpsL | ccactccgaalaagcc |
| SEQ ID NO 37 | rpsL | cactcclaagaagcc |
| SEQ ID NO 38 | rpsL | gggtgaaggacctg |
| SEQ ID NO 39 | rpsL | gggtgaaglacctg |
| SEQ ID NO 40 | rpsL | gggtlaaggacctg |
| SEQ ID NO 41 | Kan | gtcacgtcatgaaagt |
| SEQ ID NO 42 | Kan | glcacttcatgaaagt |
| SEQ ID NO 43 | Kan | gtlacgtcatgaaagt |
| SEQ ID NO 44 | Kan | gtcacgtcatgaaa |
| SEQ ID NO 45 | Kan | gtcacltcatgaaa |
| SEQ ID NO 46 | Kan | gllacgtcatgaaa |
| SEQ ID NO 47 | Kan | gacgaagtcgtaacaa |
| SEQ ID NO 48 | Kan | gacgaaltcgtaacaa |
| SEQ ID NO 49 | Kan | galgaagtcgtaacaa |
| SEQ ID NO 50 | gyrA | gcgtcgatctacga |
| SEQ ID NO 51 | gyrA | gcgtlgatctacga |
| SEQ ID NO 52 | gyrA | gcltcgatctacga |
| SEQ ID NO 53 | gyrA | cgtcgatctacgaca |
| SEQ ID NO 54 | gyrA | cgtlgatctacgaca |
| SEQ ID NO 55 | gyrA | cgtcgatltacgaca |
| SEQ ID NO 56 | rpoB | ccaattcatggtcca |
| SEQ ID NO 57 | rpoB | ccaattcatlglcca |
| SEQ ID NO 58 | rpoB | ccaattcatggtcta |
| SEQ ID NO 59 | rpoB | ccaattcatgtacc |
| SEQ ID NO 60 | rpoB | ccaatlcatgtatc |
| SEQ ID NO 61 | rpoB | ccaattcatltacc |
| SEQ ID NO 62 | rpoB | ggggttgacctacaagc |
| SEQ ID NO 63 | rpoB | ggggttgacctalaagc |
| SEQ ID NO 64 | rpoB | gggttgalctacaagc |
| SEQ ID NO 65 | rpoB | cggggttgaccgacaa |
| SEQ ID NO 66 | rpoB | cggggttgaccgataa |
| SEQ ID NO 67 | rpoB | cggggttgalcgacaa |
| SEQ ID NO 68 | rpoB | cggggttgacccgcaa |
| SEQ ID NO 69 | rpoB | cggggttgacccgtaa |
| SEQ ID NO 70 | rpoB | cggggttgaclcgcaa |
| SEQ ID NO 71 | rpoB | cgactgtlggcgctg |
| SEQ ID NO 72 | rpoB | cgactgttglcgctg |
| SEQ ID NO 73 | rpoB | cgactlttggcgctg |
| SEQ ID NO 74 | rpoB | cgactgtgggcgctg |
| SEQ ID NO 75 | rpoB | cgactgtggtcgctg |
| SEQ ID NO 76 | rpoB | cgactltgggcgctg |
| SEQ ID NO 77 | kalG | gatcaccaccggc |
| SEQ ID NO 78 | kalG | gatcaccacclgc |
| SEQ ID NO 79 | kalG | gatcaclaccggc |
| SEQ ID NO 80 | kalG | gatcaccacaggca |
| SEQ ID NO 81 | kalG | gatcaccacaglca |
| SEQ ID NO 82 | kalG | gatcaclacaggca |
| SEQ ID NO 83 | inhA | gaggcgataggttgt |
| SEQ ID NO 84 | inhA | gaggctataggttgt |
| SEQ ID NO 85 | inhA | gatgcgataggttgt |
| SEQ ID NO 86 | inhA | gagatgataggttgt |
| SEQ ID NO 87 | inhA | gagagataggttgt |
| SEQ ID NO 88 | inhA | gatatgataggttgt |
| SEQ ID NO 89 | inhA | agacgataggctgtc |
| SEQ ID NO 90 | inhA | agacgataggctltc |
| SEQ ID NO 91 | inhA | agacgatatgctgtc |
| SEQ ID NO 92 | inhA | gagacgataggatgt |
| SEQ ID NO 93 | inhA | gagacgataggattt |
| SEQ ID NO 94 | inhA | gagacgatatgatgt |

Schließlich betrifft die vorliegende Erfindung auch ein Verfahren zum Nachweis von Tuberkulose-Erregern bzw. Mykobakterien bzw. deren DNA, umfassend die folgenden Schritte:
- Bereitstellen einer Probe aus einem menschlichen oder tierischen Patienten, enthaltend Mykobakterien bzw. gegebenenfalls enthaltend Mykobakterien,
- Inkubieren der Probe mit eine Base,
- Isolieren von Mykobakterien-DNA aus den Mykobakterien,
- Amplifizieren der isolierten Mykobakterien-DNA, vorzugsweise mittels Polymerase-Ketten-Reaktion (PCR), wobei vorzugsweise mehr als 30 Amplifikationszyklen durchgeführt werden,
- Nachweisen der amplifizierten Mykobakterien-DNA.

Bezüglich weiterer Merkmale und Vorteile zu dem Verfahren, insbesondere bezüglich seiner Verwendungsmöglichkeiten, wird ebenfalls vollständig auf die bisherige und die noch folgende Beschreibung Bezug genommen.

Weitere Merkmale der Erfindung ergeben sich aus der nachfolgenden Beschreibung von Figuren und Beispielen sowie aus den Unteransprüchen in Kombination mit den Figuren. Hierbei können die verschiedenen Merkmale jeweils für sich oder in Kombination miteinander verwirklicht sein.

In den Abbildungen zeigen
- Fig. 1:: Beispiel eines erfindungsgemäßen Teststreifens mit verschiedenen erfindungsgemäßen Oligonukleotid-Sonden.
- Fig. 2:: Mögliche Testergebnisse auf einem erfindungsgemäßen Teststreifen.

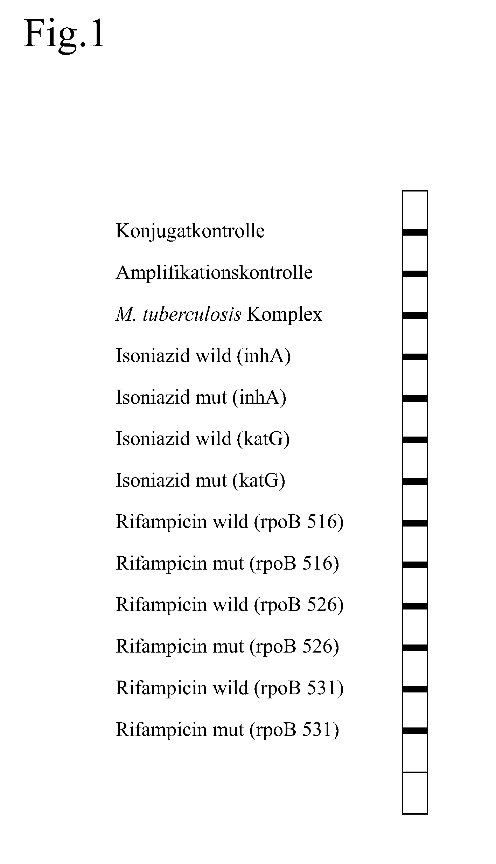

Fig. 1 zeigt ein Beispiel eines möglichen Aufbaus eines erfindungsgemäßen Teststreifens mit entsprechenden Oligonukleotid-Sonden, auf denen die aufgearbeiteten DNA-Amplifikate aufgetragen werden können, sodass diese mit den entsprechenden Sondensequenzen interagieren. Zu erkennen sind zum einen die Konjugatkontrolle und die Amplifikationskontrolle (GAP-DH). Ferner sind einzelne Reaktionszonen mit Oligonukleotid-Sonden zum Nachweis des Mycobacterium tuberculosis-Komplexes sowie der Gene, die Resistenzen gegenüber den Antibiotika Isoniazid und Rifampicin kennzeichnen, zu erkennen. Speziell können hier Gene, die Resistenzen gegenüber den Wildtypen und den Mutationen kennzeichnen, nachgewiesen werden. So sind hier im Einzelnen gezeigt: Isoniazid wild (inhA), Isoniazid mut (inhA), Isoniazid wild (katG), Isoniazid mut (katG), Rifampicin wild (rpoB 516), Rifampicin mut (rpoB 516), Rifampicin wild (rpoB 526), Rifampicin mut (rpoB 526) Rifampicin wild (rpoB 531) und Rifampicin mut (rpoB 531). Ein derartiger Teststreifen erlaubt die nähere Charakterisierung einer Tuberkuloseinfektion, gegebenenfalls einer Tuberkuloseerkrankung.
- Konjugatkontrolle:
   Diese Reaktionszone dokumentiert die Effizienz der Konjugatbindung. Sie muss immer entwickelt sein.
- Amplifikationskontrolle:
   Diese Reaktionszone enthält eine Sonde für ein Gen, das in jeder humanen DNA-Probe zu finden ist, beispielsweise das "housekeeping Gen" GAP-DH. Sie dient als Kontrolle der DNA-Isolierung, DNA-Amplifikation und der Hybridisierung. Die Reaktionszone ist nicht entwickelt, wenn die Probe aus einer Bakterienkultur stammt.
- Mycobacterium tuberculosis Komplex:
   Diese Reaktionszone ist entwickelt, wenn DNA von Erregern des Mycobacterium tuberculosis Komplexes in der Probe vorhanden ist.
- Isoniazid wild (inhA und katG):
   Diese Reaktionszonen sind nur entwickelt, wenn unmodifizierte inhA- bzw. katG-Gene (Wildtyp) in der Probe sind. Das bedeutet, der Mykobakterien-Stamm zeigt Merkmale einer Empfindlichkeit gegen Isoniazid.
- Isoniazid mut (inhA):
   Diese Reaktionszone ist nur entwickelt, wenn mindestens eine der folgenden Mutationen im inhA-Gen vorliegen, die für eine Resistenz gegenüber Isoniazid verantwortlich sind:
      inhA-16 A → G
      inhA-15 C → T
      inhA-8 T → A
      inhA-8 T → C
- Isoniazid mut (katG):
   Diese Reaktionszone ist nur entwickelt, wenn die folgende Mutation im katG-Gen vorliegt, die für eine Resistenz gegenüber Isoniazid verantwortlich ist:
      315S → T
- Rifampicin wild (rpoB 516, rpoB 526, rpoB 531):
   Diese Reaktionszonen sind nur entwickelt, wenn unmodifizierte rpoB-Gene (Wildtyp) in der Probe sind. Das bedeutet, der Mykobakterien-Stamm zeigt Merkmale einer Empfindlichkeit gegen Rifampicin.
- Rifampicin mut (rpoB 516, rpoB 526, rpoB 531):
   Diese Reaktionszonen sind nur entwickelt, wenn mindestens eine der folgenden Mutationen des rpoB-Gens in der Probe vorliegt, die für eine Resistenz gegenüber Rifampicin verantwortlich sind:
      rpoB 516D → V
      rpoB 516 D → Y
      rpoB 526 H → Y
      rpoB 526 H → D
      rpoB 526 H → R
      rpoB 531 S → L
      rpoB 531 S → W
   Das Ausbleiben mindestens einer Wildtyp-Reaktionszone deutet auf eine Resistenz gegenüber dem entsprechenden Antibiotikum hin.
   Wenn sowohl die Wildtyp-Reaktionszone als auch die Mutations-Reaktionszone eines bestimmten Zielgens entwickelt sind, deutet dies auf ein gemischtes Vorkommen sowohl empfindlicher (sensibler) als auch resistenter Mycobacterium tuberculosis-Stämme hin.

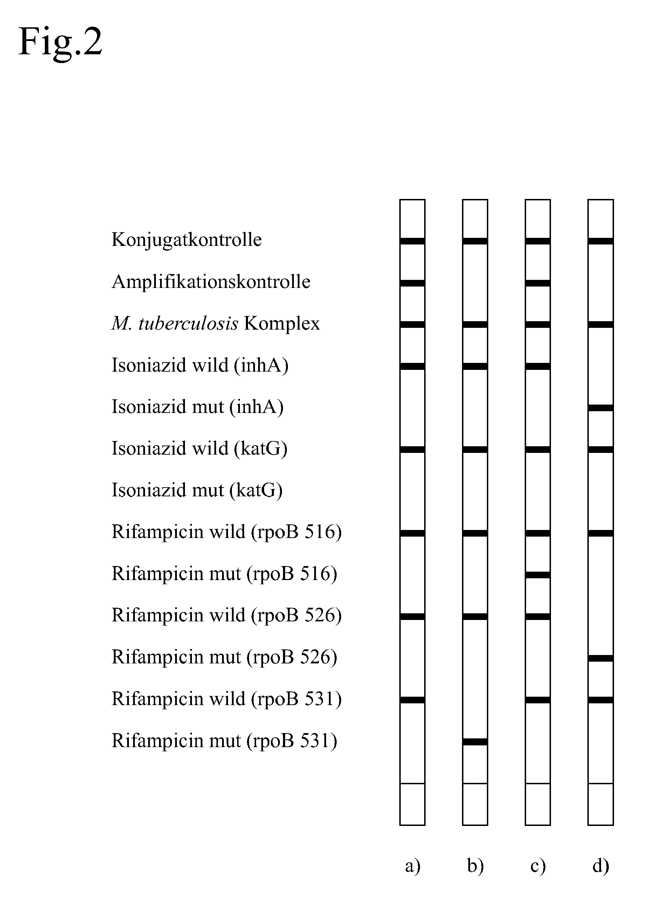

Fig. 2 zeigt ein mögliches Testergebnis.

In Fig. 2a wird ein Ergebnis gezeigt, bei dem der Mycobacterium tuberculosis-Komplex und alle Wildtypgene nachgewiesen werden. Die Erreger zeigen keine Merkmale einer Resistenz gegenüber den aufgeführten Antibiotika.

Fig. 2b zeigt ein mögliches Testergebnis bei dem der Mycobacterium tuberculosis-Komplex und alle Wildtypgene außer rpoB 531 nachgewiesen werden. Stattdessen ist die rpoB 531 Mutations-Sonde entwickelt. Die Erreger zeigen Merkmale einer Resistenz gegenüber Rifampicin. Die Amplifikationskontrolle fehlt. Bei Proben, die aus einer Bakterienkultur stammen, entwickelt sich die Amplifikationskontrollzone nicht.

Fig. 2c zeigt ein mögliches Testergebnis, bei dem der Mycobacterium tuberculosis-Komplex und alle Wildtypgene nachgewiesen werden. Zusätzlich ist die rpoB 516 Mutations-Sonde entwickelt. Wenn sowohl die Wildtyp-Reaktionszone als auch die Mutations-Reaktionszone eines bestimmten Zielgens entwickelt sind, deutet dies auf ein gemischtes Vorkommen sowohl empfindlicher (sensibler) als auch resistenter Mycobacterium tuberculosis Stämme hin.

Fig. 2d zeigt ein mögliches Testergebnis, bei dem der Mycobacterium tuberculosis-Komplex und alle Wildtypgene außer inhA und rpoB 526 nachgewiesen werden. Stattdessen sind die inhA und die rpoB 526 Mutations-Sonden entwickelt. Die Erreger zeigen Merkmale einer Resistenz gegenüber den Erstrangmedikamenten Isoniazid und Rifampicin. Stämme, die mindestens gegen Isoniazid und Rimfampicin resistent sind, werden als multresistente Tuberkulosestämme bezeichnet. Die Amplifikationskontrolle fehlt. Bei Proben, die aus einer Bakterienkultur stammen, entwickelt sich die Amplifikationskontrollzone nicht.

### Beispiele

1. Nachweis von Tuberkulose-DNA sowie von Tuberkuloseresistenzen Probenaufarbeitung :
1. Eine Sputum-Probe wurde in einem geeigneten Behältnis zur Verfügung gestellt, in dem die Mykobakterien-DNA intakt blieb.
2. Die Sputum-Probe wurde mit einer NALC-NaOH-Lösung bei Raumtemperatur und während eines Zeitraums von 15 min. inkubiert.
3. Nach der Inkubation wurde die Mykobakterien-DNA aus den Mykobakterien aufgeschlossen.
4. Die mittels zellulären Aufschluss freigesetzte Mykobakterien-DNA wurde mit Hilfe einer DNA-Reinigungssäule (z.B. Qiagen QIAamp®-DNA-Mini-Kit) isoliert.
5. Die Reaktionsgemische für die Polymerase-Kettenreaktion (PCR) wurden in einem Raum pipettiert, der getrennt von einem Thermocycler und frei von amplifizierter DNA war. Für die Pipettierschritte, die zügig durchgeführt wurden, wurden DNAase freie Reaktionsgefäße sowie sterile Pipettenspitzen mit Filtereinsatz verwendet.
Die Amplifikationsansätze enthielten:

| | |
|---|---|
| PN-Mix (biotinylierte Primer, ausgewählt aus der Gruppe bestehend aus SEQ ID No 1 bis 10 und Kombinationen davon) | 15 µL |
| 10x Polymerase Puffer | 2,5 µL |
| MgCl₂-Lösung (Endkonzentration 1,0 mM MgCl₂) Thermostabile DNA Polymerase | 1 Unit |
| Proben DNA (ca. 150-300 ng) | γ µL |
| H₂O | z µL |
| Endvolumen | 25 µL |

Um Pipettierschritte einzusparen und ungenaues Pipettieren als Fehlerquellen auszuschließen, wurde bei mehreren DNA-Proben eine Mastermischung aus PN-Mix, Puffer, MgCl₂-Lösung, DNA Polymerase und ggf. Wasser hergestellt. Diese Mastermischung wurde in PCR-Reaktionsgefäßen aufgeteilt. Anschließend wurde die DNA hinzugegeben.
6. Programmierungsprotokoll für den Thermocycler

| | | | |
|---|---|---|---|
| 5 | min. | 95°C | 1 Zyklus |
| 30 | sec. | 95°C | 14 Zyklen |
| 2 | min. | 60°C | |
| 10 | sec. | 95°C | 36 Zyklen |
| 30 | sec. | 55°C | |
| 30 | sec. | 72°C | |
| 8 | min. | 72°C | 1 Zyklus |
| | 4°C | | ∞ |

Nachweis und Charakterisierung der PCR-Fragmente:
- Agarose-Gelektrophorese:
   Zur Auftrennung der PCR-Produkte wurden von jedem PCR-Ansatz 5 µL auf ein zweiprozentiges Agarosegel aufgetragen. Es war dabei nicht notwendig, die Proben für die Elektrophorese noch zusätzlich mit Gel-Ladepuffer zu mischen.
   Die Amplifikations-Produkte hatten Längen zwischen 210 bp und 372 bp.
   Die mitgelieferte humane DNA diente als Amplifikationskontrolle für die PCR. Bei einer anschließenden Hybridisierung entwickelte sich die Amplifikationsbande sowie die Konjugatkontrolle.

Bei der Charakterisierung und Differenzierung der PCR-Fragmente mittels reverser Hybridisierung mit einem erfindungsgemäßen Kit, war es nicht notwendig, die hergestellten PCR-Produkte in einer Agarose-Gelektrophorese zu analysieren.

Das verwendete Kit wies die folgenden Komponenten auf:

| | |
|---|---|
| Nitrocellulose-Streifen, beschichtet mit erfindungsgemäßen Oligonukleo tidsonden | 12 Streifen |
| Denaturierungsreagenz | 0,6 mL |
| Hybridisierungspuffer | 15 mL |
| Stringent-Waschlösung | 40 mL |
| Rinse, 5x konzentriert | 20 mL |
| Streptavidin gekoppelte alkalische Phosphatase-Konzentrat | 0,2 mL |
| Konjugat-Puffer | 15 mL |
| NBT/BCIP | 15 mL |
| Inkubationswanne | 1 |

Die Reagenzien der Kit-Packung enthielten als Konservierungsmittel und zum Schutz vor mikrobiellem Wachstum 0, 1 % Natrium-Azid.

Vorbereitung der Reagenzien:
- Verdünnung des Streptavidin gekoppelten alkalischen Phosphatase-Konzentrats (im Folgenden als Konjugat-Konzentrat bezeichnet): Das Konjugat-Konzentrat wurde kurz vor Gebrauch im Verhältnis 1:100 mit Konjugat-Puffer verdünnt, d.h. pro Strip wurden 10 µL Konjugat-Konzentrat in 1 mL Konjugat-Puffer verdünnt und sorgfältig gemischt.
- Verdünnung des Rinse-Konzentrats: Die 5x konzentrierte Rinse-Lösung wurde vor Gebrauch im Verhältnis 1:5 mit deionisiertem Wasser verdünnt, d.h. auf einen Teil 5x konzentrierte Rinse-Lösung kamen 4 Teile Wasser.

### Testdurchführung

1. Die Pufferlösungen wurden auf 47 °C vorgewärmt, alle anderen Lösungen auf Raumtemperatur angewärmt. Inkubationswannen entsprechend der Anzahl der Proben und die Kontrollen wurden vorbereitet. Vertiefungen, die benutzt werden sollten, wurden am Rand markiert. Bereits gebrauchte Kavitäten wurden nicht noch einmal verwendet.
2. In die markierten Kavitäten wurden 40 Mikroliter Denaturierungsreagenz pipettiert.
3. Je 20 Mikroliter Amplifikat von jedem der zu untersuchenden PCR-Ansätze wurden zu dem Tropfen Denaturierungsreagenz pipettiert, gut gemischt und 5 Minuten bei Raumtemperatur inkubiert.
4. Es wurde jeweils vorsichtig 1 Milliliter vorgewärmter und gemischter Hydridisierungspuffer hinzu gegeben.
5. Die einzelnen Streifen wurden mit einer Pinzette aus dem Röhrchen entnommen und in die Inkubationswanne gelegt. Die Streifen wurden vollständig mit Flüssigkeit bedeckt. Die beschichtete Seite wies nach oben. Dies galt auch für alle nachfolgenden Inkubations- und Waschschritte. Streifen, die sich zum Beispiel durch Zugabe von Waschpuffer wendeten, wurden mit einer Pinzette an einem Ende aufgenommen und zurück gedreht.
6. Die Wanne wurde für 30 Minuten bei 47 °C im Schüttelwasserbad inkubiert (Frequenz beachten).
7. Der Hybridisierungspuffer wurde vollständig abgegossen und die Streifen anschließend zweimal 1 Minute mit je 1 Milliliter vorgewärmter und gemischter Stringent-Waschlösung gewaschen.
8. Jeweils 1 Milliliter vorgewärmte Stringentwaschlösung wurde zugegeben und 15 Minuten bei 47 °C im Wasserbad unter Schütteln inkubiert.
9. Von diesem Schritt an wurde bei Raumtemperatur gearbeitet. Die Lösung wurde abgeklopft und die Streifen zweimal mit je 1 Mililiter verdünnter Rinse-Lösung je 1 Minute gewaschen (Schüttler).
10. 1 Milliliter vorbereitetes Konjugat (Konjugatkonzentrat im Verhältnis 1:100 mit Konjugat Puffer verdünnt) wurde zu jedem Streifen gegeben und 30 Minuten auf dem Horizontalschüttler inkubiert.
11. Das Konjugat wurde entfernt und dreimal je 1 Minute mit je 1 Milliliter verdünnter Rinse-Lösung gewaschen (Horizontalschüttler).
12. Je 1 Milliliter auf Raumtemperatur erwärmtes Substrat wurde in die Kavitäten pipettiert und, je nachdem wie rasch die Farbreaktion ablief, etwa 10 Minuten auf dem Horizontalschüttler inkubiert.
13. Die Reaktion wurde durch zweimaliges Waschen mit destilliertem Wasser gestoppt. Die Streifen wurden mit einer Pinzette aus den Kavitäten genommen und zum Trocknen auf Filterpapier gelegt. Die getrockneten Streifen wurden anschließend ausgewertet und lichtgeschützt aufbewahrt .

Mittels des vorstehend beschriebenen Verfahrens konnte die in der Sputum-Probe enthaltene Mykobakterien-DNA erfolgreich nachgewiesen werden.

Darüber hinaus ließen sich mittels der Erfindung auch die in den im Folgenden dargestellten Tabellen 3 bis 5 aufgeführten Antibiotika-Resistenzen in von Patienten stammenden Proben erfolgreich nachweisen.

**Tabelle 3: Rifampicin-Resistenzen**

| **Zielregion** | **Mutation** | **Aminosäureaustausch** | **Sonde** |
|---|---|---|---|
| rpoB 516 | GAC -> GTC | D516V | SEQ ID NO 56 |
| rpoB 516 | GAC -> TAC | D516Y | SEQ ID NO 59 |
| rpoB 526 | CAC -> TAC | H526Y | SEQ ID NO 62 |
| rpoB 526 | CAC -> GAC | H526D | SEQ ID NO 65 |
| rpoB 526 | CAC -> CGC | H526R | SEQ ID NO 68 |
| rpoB 531 | TCG -> TTG | S531 L | SEQ ID NO 71 |
| rpoB 531 | TCG -> TGG | S531 W | SEQ ID NO 74 |

**Tabelle 4: Isoniazid-Resistenzen**

| **Zielregion** | **Mutation** | **Aminosäureaustausch** | **Sonde** |
|---|---|---|---|
| katG 315 | AGC -> ACC | S315T | SEQ ID NO 77 |
| katG 315 | AGC -> ACA | S315T | SEQ ID NO 80 |

**Tabelle 5: weitere Isoniazid-Resistenzen**

| **Zielregion** | **Mutation** | **Sonde** |
|---|---|---|
| inhA-16 | A -> G | SEQ ID NO 83 |
| inhA-15 | C -> T | SEQ ID NO 86 |
| inhA-8 | T -> C | SEQ ID NO 89 |
| inhA-8 | T -> A | SEQ ID NO 92 |

### 2. Sensitivität des erfindungsgemäßen Verfahrens bzw. Kits

### 2.1 erste klinische Untersuchung

Mittels des erfindungsgemäßen Verfahrens bzw. Kits wurden 31 Proben aus respiratorischem Material (Sputum, Bronchoalveoläre-Lavage, Tracheobronchoskopie), welche unter dem Mikroskop zu positiven Ergebnissen führten (Anwesenheit von Mykobakterien), untersucht. Hierbei konnte bei 29 Proben das mikroskopisch positive Ergebnis bestätigt werden. Dies entsprach insgesamt einer Bestätigung der mikroskopisch positiven Ergebnisse von 93,5 %. Weiterhin wurden auch Proben, welche unter dem Mikroskop negative Ergebnisse lieferten, mittels des erfindungsgemäßen Verfahrens bzw. Kits untersucht. Insgesamt wurden 13 derartiger Proben untersucht. Hierbei konnten bei 9 Proben Mykobakterien trotz eines mikroskopisch negativen Ergebnisses nachgewiesen werden. Dies entspricht insgesamt einem Nachweis von Mykobakterien bei 69.2 % der mikroskopisch negativen Proben. Dies zeigt die erhöhte Sensitivität des erfindungsgemäßen Verfahrens bzw. Kits gegenüber dem konventionellen mikroskopischen Nachweis von Mykobakterien.

### 2.2 zweite klinische Untersuchung

In einer ersten Studie wurden Sputum-Proben von 55 Patienten mit einer NALC-NaOH-Lösung vorbehandelt bzw. dekontaminiert. Anschließend wurde die Mykobaterien-DNA aus den Sputumproben extrahiert und mittels PCR amplifiziert, wobei insgesamt 50 Amplifikationszyklen gefahren wurden. Nach Amplifikation der Mykobakterien-DNA wurden die Amplifikate aufgetrennt und mittels reverser Hybridisierung unter Verwendung von Teststreifen, wie sie beispielhaft in den Figuren 1 und 2 dargestellt und in den entsprechenden Figurenbeschreibung erläutert sind, nachgewiesen. Konkret wurden hierzu Rifampicin/Isoniazid (RMP/INH)-Teststreifen, Chinolon-Teststreifen und Aminoglykosid-Teststreifen verwendet. Für die Amplifikation der Mykobakterien-DNA wurden erfindungsgemäße Primer eingesetzt. Für den Nachweis der Amplifikate mittels reverser Hybridisierung wurden erfindungsgemäße Olignucleotid-Sonden verwendet.

Von den 55 Sputum-Proben waren 35 Proben unter dem Mikroskop positiv auf Tuberkulose-DNA (und damit auf Tuberkulose-Erreger) getestet worden. Dieses Ergebnis konnte bei der Überprüfung dieser Proben mittels des erfindungsgemäßen Verfahrens unter Verwendung von RMP/INH-Teststreifen und Chinolon-Teststreifen jeweils verifiziert werden (100%). Bei der Verwendung von Aminoglykosid-Teststreifen zum Nachweis der Amplifikate konnten 34 von 35 der unter dem Mikroskop positiven Proben bestätigt werden (entspricht 97.14 % der unter dem Mikroskop positiven Proben).

Bei den übrigen 20 Sputum-Proben war der Nachweis auf Tuberkulose-DNA (und damit auf Tuberkulose-Erreger) unter dem Mikroskop negativ ausgefallen. Eine Überprüfung dieser Proben mittels des erfindungsgemäßen Verfahrens unter Verwendung von RMP/INH-Teststreifen zum Nachweis der DNA-Amplifikate führte bei 12 Proben zu einem positiven, d.h. das Vorliegen von Mykobakterien-DNA bestätigenden, Testergebnis (entspricht 60 % der unter dem Mikroskop als negativ beurteilten Testergebnisse). Wurden anstelle der RMP/INH-Teststreifen Chinolon-Teststreifen zum Nachweis der Amplifikate eingesetzt, so konnte bei 9 Proben die Anwesenheit von Mykobakterien-DNA und damit von Mykobakterien nachgewiesen werden (entspricht 45 % der unter dem Mikroskop als negativ beurteilten Proben). Bei der Verwendung von Aminoglykosid-Teststreifen konnten ebenfalls 9 Proben positiv auf Mykobakterien-DNA und damit auf Mykobakterien getestet werden (entspricht 45 % der unter dem Mikroskop als negativ beurteilten Proben).

Insgesamt konnten somit von den 55 Sputum-Proben bei Anwendung des erfindungsgemäßen Verfahrens unter Verwendung von RMP/INH-Teststreifen 47 der Proben positiv auf Mykobakterien-DNA und damit auf Mykobakterien getestet werden (entspricht einem Anteil von 84.45 %). Bei Anwendung des erfindungsgemäßen Verfahrens unter Verwendung von Chinolon-Teststreifen konnten insgesamt 44 der 55 Proben positiv auf Mykobakterien-DNA und damit auf Mykobakterien getestet werden (entspricht einem Anteil von 79.99%). Bei Anwendung des erfindungsgemäßen Verfahrens unter Verwendung von Aminoglykosid-Teststreifen konnten insgesamt 43 der 55 Proben positiv auf Mykobakterien-DNA und damit auf Mykobakterien getestet werden (entspricht einem Anteil von 78.18%).

In einer zweiten Untersuchung wurden 15 weitere Sputum-Proben, in welchen unter dem Mikroskop keine Mykobakterien-DNA nachgewiesen werden waren, mittels des erfindungsgemäßen Verfahrens überprüft. Bei Verwendung von RMP/INH-Teststreifen konnten bei 11 Proben die Anwesenheit von Mykobakterien-DNA nachgewiesen werden (entspricht einem Anteil von 73.33 %). Bei Verwendung von Chinolon-Teststreifen konnten 14 der 15 Proben positiv auf Mykobakterien-DNA gestestet werden (entspricht einem Anteil von 93.33 %). Bei Verwendung von Aminoglykosid-Teststreifen konnten 11 der 15 Proben positiv auf Mykobakterien-DNA getestet werden (entspricht einem Anteil von 73.33 %).

In einer dritten Untersuchung wurden 34 weitere Sputum-Proben, welche unter dem Mikroskop negativ auf Mykobakterien-DNA getestet worden waren, mittels des erfindungsgemäßen Verfahrens überprüft. Die Verwendung von RMP/INH-Teststreifen führte bei 29 Proben zu einem in Bezug auf die Anwesenheit von Mykobakterien-DNA positiven Testergebnis (entspricht einem Anteil von 85.29 %). Bei Verwendung von Chinolon-Teststreifen konnten 31 der 34 Proben positiv auf Mykobakterien-DNA getestet werden (entspricht einem Anteil von 91.18 %). Bei Verwendung von Aminoglykosid-Teststreifen wurden 25 der 34 Proben positiv auf Mykobakterien-DNA getestet (entspricht einem Anteil von 73.53%).

Von den insgesamt 69 unter dem Mikroskop negativen Sputum-Proben, welche Gegenstand der vorstehend beschriebenen drei Untersuchungen waren, konnten somit mittels des erfindungsgemäßen Verfahrens bei Verwendung von RMP/INH-Teststreifen 52 Proben positiv auf Mykobakterien-DNA (entspricht einem Anteil von 75.36 %), bei Verwendung von Chinolon-Teststreifen 54 Proben positiv auf Mykobakterien-DNA (entspricht einem Anteil von 78.26 %) und bei Verwendung von Aminoglykosid-Teststreifen 45 Proben positiv auf Mykobakterien-DNA getestet werden (entspricht einem Anteil von 65.22 %).

Insgesamt wurden in den drei Untersuchungen 104 Sputum-Proben untersucht. Von diesen konnten mittels des erfindungsgemäßen Verfahrens bei Verwendung von RMP/INH-Teststreifen 87 Proben positiv auf Mykobakterien-DNA getestet werden (entspricht einem Anteil von 83.65 %). Bei Verwendung von Chinolon-Teststreifen konnten 89 Proben positiv auf Mykobakterien-DNA getestet werden (entspricht einem Anteil von 85.58 %). Bei Verwendung von Aminoglykosid-Teststreifen konnten 79 Proben positiv auf Mykobakterien-DNA getestet werden (entspricht einem Anteil von 75.96 %).

Die mittels des erfindungsgemäßen Verfahrens positiv auf Mykobakterien-DNA getesteten Proben enthielten dabei sensible und/oder resistente Mykobakterien.

Zusammenfassend lässt sich sagen, dass das erfindungsgemäße Verfahren gegenüber dem konventionellen mikroskopischen Nachweisverfahren eine deutlich erhöhte Sensitivität aufweist. So konnte ein signifikanter Anteil der Proben, welche unter dem Mikroskop negativ waren, mittels des erfindungsgemäßen Verfahrens positiv auf Mykobakterien-DNA getestet werden. Mit anderen Worten führt der konventionelle mikroskopische Nachweis in vielen Fällen zu falsch negativen Testergebnissen, wohingegen das Risiko von falsch negativen Testergebnissen im Falle des erfindungsgemäßen Verfahrens signifikant reduziert ist.

## Patentansprüche

1. in vitro-Verfahren zum Nachweis von DNA von Tuberkulose-Erregern bzw. Mykobakterien bzw. deren DNA, umfassend die folgenden Schritte:
• Bereitstellen einer Probe, enthaltend Mykobakterien bzw. gegebenenfalls enthaltend Mykobakterien,
• Inkubieren der Probe mit einer Base,
• Isolieren von Mykobakterien-DNA aus den inkubierten Mykobakterien,
• Amplifizieren der isolierten Mykobakterien-DNA mittels Polymerase-Ketten-Reaktion (PCR), wobei mehr als 30 Amplifikationszyklen durchgeführt werden,
• Nachweisen der amplifizierten Mykobakterien-DNA.

2. in vitro-Verfahren nach Anspruch 1, **dadurch gekennzeichnet, dass** zum Amplifizieren der Mykobakterien-DNA mehr als 40, vorzugsweise 50 oder mehr als 50, Amplifikationszyklen gefahren werden.

3. in vitro-Verfahren nach Anspruch 1 oder 2, **dadurch gekennzeichnet, dass** als Probe eine Speichelprobe, Sputumprobe, Rachenabstrich, Bronchialschleimprobe, ein Bronchialbioptat und/oder Lungenbioptat, vorzugsweise eine Sputumprobe, verwendet wird.

4. in vitro-Verfahren nach einem der vorhergehenden Ansprüche, **dadurch gekennzeichnet, dass** die Probe mit einer anorganischen Base, vorzugsweise mit einer wässrigen Lösung einer anorganischen Base, inkubiert wird.

5. in vitro-Verfahren nach einem der vorhergehenden Ansprüche, **dadurch gekennzeichnet, dass** die Base ausgewählt ist aus der Gruppe bestehend aus Natriumhydroxyid, Kaliumhydroxid, Bariumhydroxid, Calciumhydroxid und Mischungen davon.

6. in vitro-Verfahren nach einem der vorhergehenden Ansprüche, **dadurch gekennzeichnet, dass** zum Amplifizieren der Mykobakterien-DNA Primer, vorzugsweise ausgewählt aus der Gruppe bestehend aus Oligonukleotide gemäß den SEQ ID NO 1 bis 10 und Kombinationen davon, eingesetzt werden.

7. in vitro-Verfahren nach einem der vorhergehenden Ansprüche, **dadurch gekennzeichnet, dass** die Mykobakterien-DNA-Amplifikate mittels Hybridisierung mit sequenzspezifischen Oligonukleotid-Sonden nachgewiesen werden.

8. in vitro-Verfahren nach einem der vorhergehenden Ansprüche, **dadurch gekennzeichnet, dass** die Mykobakterien-DNA-Amplifikate mittels Oligonukleotid-Sonden, ausgewählt aus der Gruppe bestehend aus Oligonukleotide gemäß den SEQ ID NO 11 bis 94 und Kombinationen davon, nachgewiesen werden.

9. in vitro-Verfahren nach einem der vorhergehenden Ansprüche, **dadurch gekennzeichnet, dass** es zum Nachweis von Aktivierungs- und/oder Resistenzgenen für Antituberkulotika, insbesondere Antibiotika, verwendet wird.

10. in vitro-Verfahren nach einem der vorhergehenden Ansprüche, **dadurch gekennzeichnet, dass** es zur Differenzierung zwischen sensiblen Mykobakterien und resistenten Mykobakterien, insbesondere multiresistenten und/oder extrem resistenten Mykobakterien, verwendet wird.

11. in vitro-Verfahren nach einem der vorhergehenden Ansprüche, **dadurch gekennzeichnet, dass** es zum Nachweis bzw. zur Diagnose einer Tuberkuloseinfektion, einer Tuberkuloseerkrankung und/oder von Tuberkulose-Resistenzen, vorzugsweise einer Isoniazid-Resistenz und/oder Rifampicin-Resistenz, verwendet wird.

12. Oligonukleotid-Primer, insbesondere zum Amplifizieren von Mykobakterien-DNA, ausgewählt aus der Gruppe bestehend aus Oligonukleotide gemäß der SEQ ID NO 1 bis 10 und Kombinationen davon.

13. Oligonukleotid-Sonden, insbesondere zum Nachweisen, insbesondere Identifizieren und vorzugsweise Differenzieren, von Mykobakterien-DNA-Amplifikaten, ausgewählt aus der Gruppe bestehend aus Oligonukleotide gemäß den SEQ ID NO 11 bis 94 und Kombinationen davon.

14. Träger zum Nachweisen, insbesondere Identifizieren und vorzugsweise Differenzieren, von Mykobakterien-DNA-Amplifikaten, vorzugsweise in Form eines Teststreifens, umfassend Oligonukleotid-Sonden, ausgewählt aus der Gruppe bestehend aus Oligonukleotide gemäß den SEQ ID NO 11 bis 94 und Kombinationen davon.

15. Kit, insbesondere zur Verwendung in einem Verfahren nach einem der Ansprüche 1 bis 11, zumindest umfassend eine der folgenden Komponenten:
• Oligonukleotid-Primer, ausgewählt aus der Gruppe bestehend aus Oligonukleotide gemäß den SEQ ID NO 1 bis 10 und Kombinationen davon und/oder
• Oligonukleotid-Sonden, ausgewählt aus der Gruppe bestehend aus Oligonukleotide gemäß den SEQ ID NO 11 bis 94 und Kombinationen davon.
